# EUROPÄISCHE PATENTANMELDUNG

(11) **EP 4 424 330 A1**
(43) Veröffentlichungstag der Anmeldung: **04.09.2024**
(21) Anmeldenummer: 23159674.3
(22) Anmeldetag: 02.03.2023
(51) Int. Cl.: A61L 2/08

(54) **VERFAHREN ZUM SERIELLEN TRANSFER VON GEBINDEN DURCH EINE PROZESSKAMMER ZUR WEITERVERARBEITUNG IN EINEM CONTAINMENT UNTER ASEPTISCHEN BEDINGUNGEN UND PRODUKTIONSANLAGE DAZU**

(71) Anmelder: SKAN Stein AG, 4332 Stein (CH)
(72) Erfinder: Alava, Andreas, 4055 Basel (CH); Bielmann, Michael, 8800 Thalwil (CH); Lehmann, Frank, 4102 Binningen (CH); Seuret, Dominique, 4132 Muttenz (CH)
(74) Vertreter: Ullrich, Gerhard

(57) **Zusammenfassung**

Das erfindungsgemässe Verfahren mit dazu konzipierter Produktionsanlage (**1**) ist zum seriellen Transfer von Gebinden (**9**) durch eine Prozesskammer (**3**) zur Weiterverarbeitung in einem Containment (**5**) unter aseptischen Bedingungen bestimmt. Dies mittels einer in der Prozesskammer (**3**) installierten Elektronen-Strahlungsquelle (**7**), welche ein Austrittsfenster (**70**) besitzt, aus dem ein Emissionsfeld von Elektronen austritt. Das einzelne Gebinde (**9**) weist ein Behältnis (**94**) auf, das mit einer Abdeckung (**98**) verschlossen ist. Der Prozesskammer (**3**) ist eine Bereitstellungsstation (**2**) vorgelagert, die zusammen mit dem Containment (**5**) eine in einem Aufstellungsraum (**A**) errichtete Produktionsanlage (**1**) bilden. Aus der Bereitstellungsstation (**2**) werden die Gebinde (**9**) einzeln und nacheinander auf eine in der Prozesskammer (**3**) installierte Fördereinrichtung (**6**) geladen. Die Fördereinrichtung (**6**) dient dazu, das jeweils aufgenommene Gebinde (**9**) durch das wirksame Emissionsfeld der Elektronen-Strahlungsquelle (**7**) zu bewegen, so dass die gesamte Oberfläche des Gebindes (**9**) dekontaminiert ist.

## Beschreibung

### Anwendungsgebiet der Erfindung

Die vorliegende Erfindung betrifft ein Verfahren zum seriellen Transfer von Gebinden durch eine Prozesskammer zur Weiterverarbeitung in einem Containment unter aseptischen Bedingungen mittels einer in der Prozesskammer installierten Elektronen-Strahlungsquelle, welche ein Austrittsfenster besitzt, aus dem ein Emissionsfeld von Elektronen austritt. Das einzelne Gebinde weist ein Behältnis mit einem aseptischen Innenraum und einem Durchlass auf, der mit einer Abdeckung verschlossen ist. Die im Innenraum gelagerten Artikel sollen nach dem Öffnen der Abdeckung in der Prozesskammer behandelt werden. Die Behältnisse der Gebinde haben z.B. die Gestalt wannenförmiger Tubs, und die Artikel sind z.B. pharmazeutische Phiolen, Vials oder Spritzen, die in systematisch angeordneten muldenförmigen Aufnahmekonturen von Nestern stecken. Der Prozesskammer ist eine Bereitstellungsstation vorgelagert, die zusammen mit dem Containment eine in einem Aufstellungsraum errichtete Produktionsanlage bilden. Aus der Bereitstellungsstation werden die Gebinde einzeln und nacheinander auf eine in der Prozesskammer installierte Fördereinrichtung geladen. Die Fördereinrichtung dient dazu, das einzeln aufgenommene Gebinde durch das wirksame Emissionsfeld der Elektronen-Strahlungsquelle zu bewegen.

### Stand der Technik

Es ist bekannt, zur Dekontamination und Sterilisation von Oberflächen in der Pharma- und Verpackungsindustrie niederenergetische Elektronenbestrahlung einzusetzen. Derartige Einrichtungen lassen sich beim Anwender mit relativ geringem apparativem Aufwand installieren und betreiben. Anlagen mit Gamma- oder Hochenergie-Elektronenstrahlen hingegen erfordern substanzielle Aufbauten und eignen sich daher nur für engere Anwenderkreise, wie z.B. zentrale Dienstleister. Anlagen zum Betrieb mit niederenergetischen Elektronen weisen Linearkathoden oder Punktquellen mit nachfolgender elektromagnetischer Aufweitung, sogenannte Scanner, auf. Eine Sterilisationsanlage mit einer Linearkathode ist z.B. in der US 8,772,743 B2 offenbart. Eine Linearkathode besteht im Wesentlichen aus einem Filament, welches als Elektronenquelle wirkt und in einer Kathode auf negativem Potential gehalten wird. Das Filament ist in einem Vakuumbehältnis angeordnet, das ein aus dünner Folie beschaffenes Elektronenaustrittsfenster aufweist. Die Elektronen werden von der Kathode in Richtung Elektronenaustrittsfenster beschleunigt und gewinnen somit genügend kinetische Energie, dieses zu durchdringen, wobei das Vakuum innerhalb des Behältnisses erhalten bleibt. Daher lässt sich der Elektronenstrahl auch bei Umgebungsdruck für bestimmte Anwendungen industriell nutzen. So lässt sich ein Behältnis, deren Oberfläche zu dekontaminieren ist, in einer Prozesskammer durch eine Elektronenwolke in einer Translationsbewegung transportieren, welche dadurch das Behältnis allseits bestrahlt. Dabei umfassen die Anlagen in der Regel mehrere Strahlungsquellen, wie z.B. mit zwei Strahlungsquellen in der US 2012/0 217 042 A1 beschrieben.

Auch beim Ein- und Ausschleusen der zu behandelnden Behältnisse in die Prozesskammer darf von der durch die Elektronenwolke erzeugten Röntgenstrahlung keine Gefährdung für die äussere Umgebung ausgehen. In den Patentpublikationen EP 2 094 313 B1, EP 2 833 928 B1, Patent EP 1 879 629 B1 und EP 3 479 848 B1 werden hierfür verschiedene Schutzbarrieren bzw. der Strahlungsrichtung ausweichende Förderstrecken vorgeschlagen.

Die Sterilisationsanlage gemäss der EP 1 685 853 B1 beruht auf nur einer Strahlungsquelle mit klarer Richtungsausprägung der erzeugten Elektronenwolke. Die Strahlungsbehandlung der gesamten Oberfläche des Behältnisses erreicht man dadurch, dass das Behältnis in einer sequenziellen Abfolge von Rotations- und Translationsbewegungen der Elektronenwolke ausgesetzt wird, dennoch verbleiben mehrere Unzulänglichkeiten. Durch die Strukturierung des Bewegungsablaufs des Behältnisses durch die Elektronenwolke in mehreren sequenziellen Etappen, ergibt sich keine eindeutige Grenze an der Elektronenquelle zwischen sterilem und unsterilem Bereich. Ferner muss auch die Halterung, welche das Behältnis trägt, im Sterilisationsprozess behandelt werden, um eine Rekontamination der am Behältnis gereinigten Oberflächen zu vermeiden, was kaum zuverlässig erreichbar ist. Schliesslich ist bei dem apparativen Aufbau die Überlagerung der Prozesskammer mit einer Laminarströmung aus steriler Luft, wodurch die sterile Prozessseite von der unsterilen Prozessseite getrennt wird, nicht realisierbar.

Die US 10,265,427 B2 hat eine Sterilisationsanlage zum Gegenstand, welche eine Strahlungsquelle, bestehend aus mehreren gerichteten Einzelquellen aufweist, deren Elektronenwolke das zu behandelnde, in sequentiellen Translations- und Rotationsbewegungen geführte Behältnis räumlich umschliesst. Die Abfolge der Sterilisation verschiedener Oberflächenareale des zu behandelnden Behältnisses in räumlich separate Unterprozesse, bietet keine Gewähr dafür, dass lückenlos alle Oberflächenareale und Manipulatoren zum Transport des Behältnisses beim Durchgang durch die Elektronenwolke sterilisiert werden. Ferner stellt sich bei diesem Anlagenkonzept beim Zuführen einer Laminarströmung aus steriler Luft in die Prozesskammer keine klare Grenze zwischen sterilem und unsterilem Bereich ein. Schliesslich erfüllt diese Anlage nicht die Ansprüche eines möglichst geringen gerätetechnischen, kompakten und kosteneffizienten Aufwands.

### Aufgabe der Erfindung

In Weiterentwicklung zum bisherigen Stand der Technik, liegt der Erfindung die Aufgabe zugrunde, ein Verfahren mit zugehöriger Produktionsanlage vorzuschlagen, womit die vollständige Oberflächendekontamination eines Behältnisses, z.B. ein wannenförmiges Tub mit in dessen Innenraum steril gelagerten Medizinprodukten, mittels einer einzelnen Elektronen-Strahlungsquelle sicher erreicht wird. Eine ergänzende Aufgabe für das Anlagenkonzept dabei ist, dass beim Einbringen einer sterilen laminaren Luftströmung in die Prozesskammer eine eindeutige Grenze zwischen sterilem und unsterilem Bereich entsteht.

### Übersicht über die Erfindung

Das Verfahren ist zum seriellen Transfer von Gebinden durch eine Prozesskammer zur Weiterverarbeitung in einem Containment unter aseptischen Bedingungen mittels einer in der Prozesskammer installierten Elektronen-Strahlungsquelle bestimmt. Aus dem Austrittsfenster der Elektronen-Strahlungsquelle tritt ein Emissionsfeld von Elektronen aus. Das einzelne Gebinde weist ein Behältnis auf, das mit einer Abdeckung verschlossen ist. Der Prozesskammer ist eine Bereitstellungsstation vorgelagert, die zusammen mit dem Containment eine in einem Aufstellungsraum errichtete Produktionsanlage bilden. Aus der Bereitstellungsstation werden die Gebinde einzeln und nacheinander auf eine in der Prozesskammer installierte Fördereinrichtung geladen. Die Fördereinrichtung dient dazu, das einzeln aufgenommene Gebinde durch das wirksame Emissionsfeld der Elektronen-Strahlungsquelle zu bewegen.

Im Arbeitsmodus der Produktionsanlage werden als aufeinanderfolgende Verfahrensschritte ausgeführt:
- Transport des äusserlich zu dekontaminierenden Gebindes durch das Emissionsfeld der Elektronen-Strahlungsquelle, während die Fördereinrichtung das aufgenommene Gebinde im Wechsel an verschiedenen Oberflächenarealen stützt, so dass die gesamte Oberfläche des Gebindes dekontaminiert ist; und
- der Transport als kombinierte Bewegung des Gebindes vor dem Austrittsfenster ausgeführt wird, nämlich:
   - als kontinuierliche oder schrittweise Vorwärtsbewegung des Gebindes und dazu gleichzeitiger Dreh- oder Taumelbewegung um sich selbst; oder
   - als schrittweise Vorwärtsbewegung des Gebindes und zwischen den vorwärtsweisenden Bewegungsschritten eine Dreh- oder Taumelbewegung um sich selbst.

Nachfolgend werden spezielle Ausführungsformen des erfindungsgemässen Verfahrens definiert:
Der kombinierten Bewegung des Gebindes vor dem Austrittsfenster, bestehend aus Vorwärtsbewegung und Dreh- oder Taumelbewegung um sich selbst, ist eine variierende seitliche Auslenkung des Gebindes überlagert, um zu erreichen, dass zumindest im Prinzip sämtliche Oberflächenareale des Gebindes während der wirksamen Bestrahlung durch die Elektronen-Strahlungsquelle zum Austrittsfenster in gleichem Abstand geführt werden.

Die Vorwärtsbewegung verläuft geradlinig.

Während des Transports bilden die Flächennormale des Austrittsfensters und die Flächennormale der Abdeckung zueinander z.B. einen Winkel von 90° oder einen Winkel zwischen 45° und maximal 135°, vorzugsweise zwischen 60° und maximal 120°.

Zur Gewährleistung des Personenschutzes im Aufstellraum gegenüber der Gefährdung, welche von der Elektronen-Strahlungsquelle im Betriebsmodus ausgeht:
- ist die Elektronen-Strahlungsquelle in kritischen Situationen mit aus der Prozesskammer heraus nach aussen offenem Strahlungsweg, zumindest auf eine unkritische Leistung herunter regelbar oder völlig deaktivierbar; oder
- bei laufend eingeschalteter Elektronen-Strahlungsquelle ist zur Abschirmung gefährlicher Strahlung der Prozesskammer eine Eingangsschleuse vorgesetzt, über welche die zu behandelnden Gebinde von der Bereitstellungsstation in die Prozesskammer eingebracht werden.

Zur Verkettung der Prozesskammer mit dem Containment ist zwischen beiden eine Abgangskammer angeordnet, über welche die in der Prozesskammer behandelten Gebinde zum Containment hin ausgeschleust werden. Die Figurenfolge 4A-4N, mit nicht durchgehend eingeschalteter Elektronen-Strahlungsquelle, zeigt die Verkettung der Prozesskammer mit dem Containment ohne eine Abgangskammer vorzusehen.

Das einzelne Gebinde hat einen aseptischen Innenraum und einen Durchlass, der mit der Abdeckung verschlossen ist, wobei im Innenraum gelagerte Artikel nach Öffnen der Abdeckung im Containment zu behandeln sind.

Das Behältnis ist z.B. ein wannenförmiges Tub. Die Abdeckung ist typischerweise auf dem den Durchlass des Behältnisses umlaufenden Rand versiegelt und bewahrt so den Innenraum des Behältnisses in sterilem Zustand. Das Behältnis weist verschiedene Oberflächenareale auf, die sich als Konturen zum Unterfassen oder klemmenden Ergreifen des Gebindes eignen. Die Artikel sind insbesondere Phiolen, Vials oder Spritzen. Von der Abdeckung bestehen zumindest Flächenanteile vorzugsweise aus einem semipermeablen Vliesgewebe, wie Tyvek^{®}.

Die Fördereinrichtung besitzt bewegliche Stützelemente, welche - während des Transports - das Gebinde im Wechsel an verschiedenen Oberflächenarealen stützend, geschaltet werden. Die Stützelemente unterfassen oder greifen einklemmend Konturen am Gebinde, z.B. am Boden des Gebindes oder setzen an dessen Behältnisrand an. In einer bevorzugten Alternative wird während des Transports das jeweilige dem Austrittsfenster zugewandte Stützelement vom Gebinde gelöst geschaltet.

Für die Bewegung der Fördereinrichtung dient ein Transportschlitten und/oder eine Hubeinrichtung.

Die Elektronen-Strahlungsquelle wird mit einer Beschleunigungsspannung zwischen 80 keV und 300 keV betrieben, vorzugsweise mit Beschleunigungsspannung zwischen 120 keV und 200 keV.

Durch die Prozesskammer fliesst eine gleichgerichtete Verdrängungsströmung, welche von oben und/oder von der Seite in die Prozesskammer eingebracht und unten und/oder seitlich aus der Prozesskammer abgeführt wird.

Nach dem Transport des Gebindes durch das Emissionsfeld der Elektronen-Strahlungsquelle und bei fliessender Verdrängungsströmung in der Prozesskammer wird eine Sterilgrenze erzielt, ab der in weiterer Prozessrichtung des Gebindes Reinraumbedingungen herrschen, z.B. die Reinraumklasse A gemäss DIN EN ISO 14644-1.

Die Produktionsanlage ist zum seriellen Transfer von Gebinden durch eine Prozesskammer zur Weiterverarbeitung in einem Containment unter aseptischen Bedingungen mittels einer in der Prozesskammer installierten Elektronen-Strahlungsquelle konzipiert. Aus dem Austrittsfenster der Elektronen-Strahlungsquelle tritt ein Emissionsfeld von Elektronen aus. Das einzelne Gebinde weist ein Behältnis auf, das mit einer Abdeckung verschlossen ist. Der Prozesskammer ist eine Bereitstellungsstation vorgelagert, die zusammen mit dem Containment die in einem Aufstellungsraum errichtete Produktionsanlage bilden. In der Prozesskammer befindet sich eine Fördereinrichtung, welche dazu bestimmt ist, die Gebinde aus der Bereitstellungsstation einzeln und nacheinander aufzunehmen und das einzeln aufgenommene Gebinde durch das wirksame Emissionsfeld der Elektronen-Strahlungsquelle zu bewegen. Die Fördereinrichtung dient zum Transport des äusserlich zu dekontaminierenden Gebindes durch das Emissionsfeld der Elektronen-Strahlungsquelle, und während des Transports stützt die Fördereinrichtung das aufgenommene Gebinde im Wechsel an verschiedenen Oberflächenarealen, so dass die gesamte Oberfläche des Gebindes dekontaminiert ist. Der Transport ist eine kombinierte Bewegung des Gebindes vor dem Austrittsfenster, nämlich:
- eine kontinuierliche oder schrittweise Vorwärtsbewegung des Gebindes und dazu gleichzeitige Dreh- oder Taumelbewegung um sich selbst; oder
- eine schrittweise Vorwärtsbewegung des Gebindes und zwischen den vorwärtsweisenden Bewegungsschritten eine Dreh- oder Taumelbewegung um sich selbst.

Nachfolgend werden spezielle Ausführungsformen der erfindungsgemässen Produktionsanlage definiert:
Der kombinierten Bewegung des Gebindes vor dem Austrittsfenster, bestehend aus Vorwärtsbewegung und Dreh- oder Taumelbewegung um sich selbst, ist eine variierende seitliche Auslenkung des Gebindes überlagert, um zu erreichen, dass zumindest im Prinzip sämtliche Oberflächenareale des Gebindes während der wirksamen Bestrahlung durch die Elektronen-Strahlungsquelle zum Austrittsfenster in gleichem Abstand geführt werden.

Die Vorwärtsbewegung ist geradlinig.

Während des Transports bilden die Flächennormale des Austrittsfensters und die Flächennormale der Abdeckung zueinander einen Winkel von z.B. 90° oder zwischen 45° und maximal 135°, vorzugsweise zwischen 60° und maximal 120°.

Zur Gewährleistung des Personenschutzes im Aufstellraum gegenüber der Gefährdung, welche von der Elektronen-Strahlungsquelle im Betriebsmodus ausgeht:
- ist die Elektronen-Strahlungsquelle in kritischen Situationen mit aus der Prozesskammer heraus nach aussen offenem Strahlungsweg, zumindest auf eine unkritische Leistung herunter regelbar oder völlig deaktivierbar; oder
- bei laufend eingeschalteter Elektronen-Strahlungsquelle ist zur Abschirmung gefährlicher Strahlung der Prozesskammer eine Eingangsschleuse vorgesetzt, über welche die zu behandelnden Gebinde von der Bereitstellungsstation in die Prozesskammer eingebracht werden.

Zur Verkettung der Prozesskammer mit dem Containment ist zwischen beiden eine Abgangskammer angeordnet, über welche die in der Prozesskammer behandelten Gebinde zum Containment hin ausgeschleust werden. Zur Verkettung der Prozesskammer mit dem Containment ohne Abgangskammer siehe Figurenfolge 4A-4N.

Das einzelne Gebinde hat einen aseptischen Innenraum und einen Durchlass, der mit der Abdeckung verschlossen ist, wobei im Innenraum gelagerte Artikel nach Öffnen der Abdeckung zur Behandlung im Containment bestimmt sind.

Das Behältnis ist z.B. ein wannenförmiges Tub. Die Abdeckung ist typischerweise auf dem den Durchlass des Behältnisses umlaufenden Rand versiegelt und bewahrt so den Innenraum des Behältnisses in sterilem Zustand. Das Behältnis weist verschiedene Oberflächenareale auf, die sich als Konturen zum Unterfassen oder klemmenden Ergreifen des Gebindes eignen. Die Artikel sind insbesondere Phiolen, Vials oder Spritzen. Von der Abdeckung bestehen vorzugsweise zumindest Flächenanteile aus einem semipermeablen Vliesgewebe, wie Tyvek^{®}.

Die Fördereinrichtung besitzt bewegliche Stützelemente, welche dazu bestimmt sind, sich während des Transports schalten zu lassen, um das Gebinde im Wechsel an verschiedenen Oberflächenarealen zu stützen. Die Stützelemente unterfassen oder greifen einklemmend Konturen am Gebinde, z.B. den Boden des Gebindes oder dessen Behältnisrand. Vorzugsweise ist während des Transports das jeweilige dem Austrittsfenster zugewandte Stützelement vom Gebinde gelöst schaltbar.

Für die Bewegung der Fördereinrichtung dient ein Transportschlitten und/oder eine Hubeinrichtung.

Die Elektronen-Strahlungsquelle wird mit einer Beschleunigungsspannung zwischen 80 keV und 300 keV betrieben, vorzugsweise mit Beschleunigungsspannung zwischen 120 keV und 200 keV.

Durch die Prozesskammer fliesst eine gleichgerichtete Verdrängungsströmung, welche von oben und/oder von der Seite in die Prozesskammer eingebracht und unten und/oder seitlich aus der Prozesskammer abgeführt wird.

Nach dem Transport des Gebindes durch das Emissionsfeld der Elektronen-Strahlungsquelle und bei fliessender Verdrängungsströmung in der Prozesskammer ist eine Sterilgrenze entstanden, ab der in weiterer Verarbeitungsrichtung des Gebindes Reinraumbedingungen herrschen, z.B. die Reinraumklasse A gemäss DIN EN ISO 14644-1.

### Kurzbeschreibung der beigefügten Zeichnungen

Es zeigen:

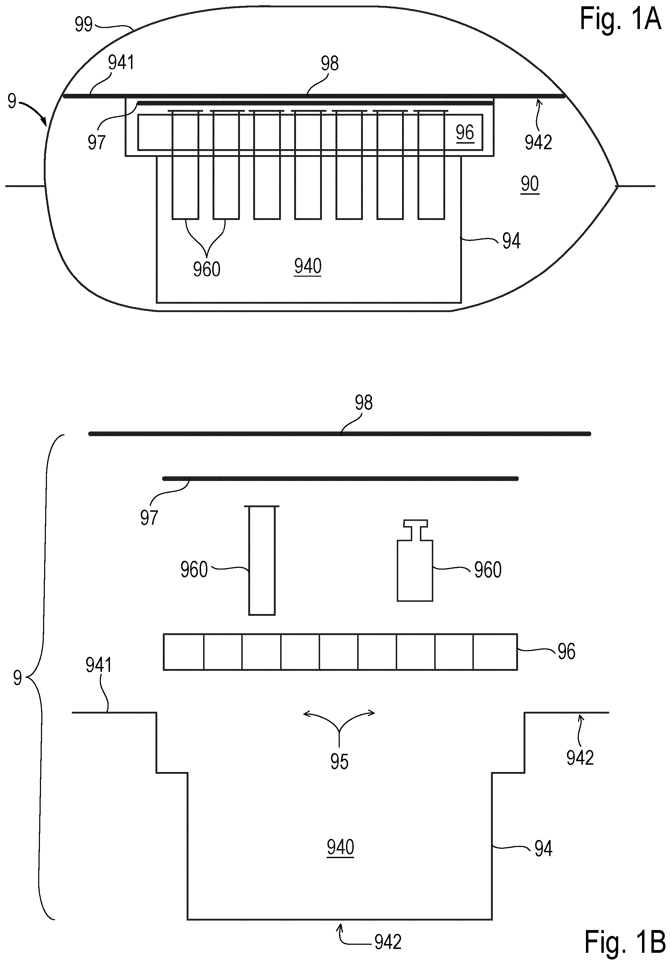

Figur 1A - ein mit dem erfindungsgemässen Verfahren in der zugehörigen Produktionsanlage zu verarbeitendes Gebinde mit geschlossener Umhüllung;
Figur 1B - das Gebinde gemäss Figur 1A ohne die Umhüllung in Explosivansicht;
Figuren 2A bis 2L: die erfindungsgemässe Produktionsanlage in einer *ersten Ausführungsform* mit auf- und abwärts führendem Bewegungsverlauf während eines vollständigen Produktionszyklus;

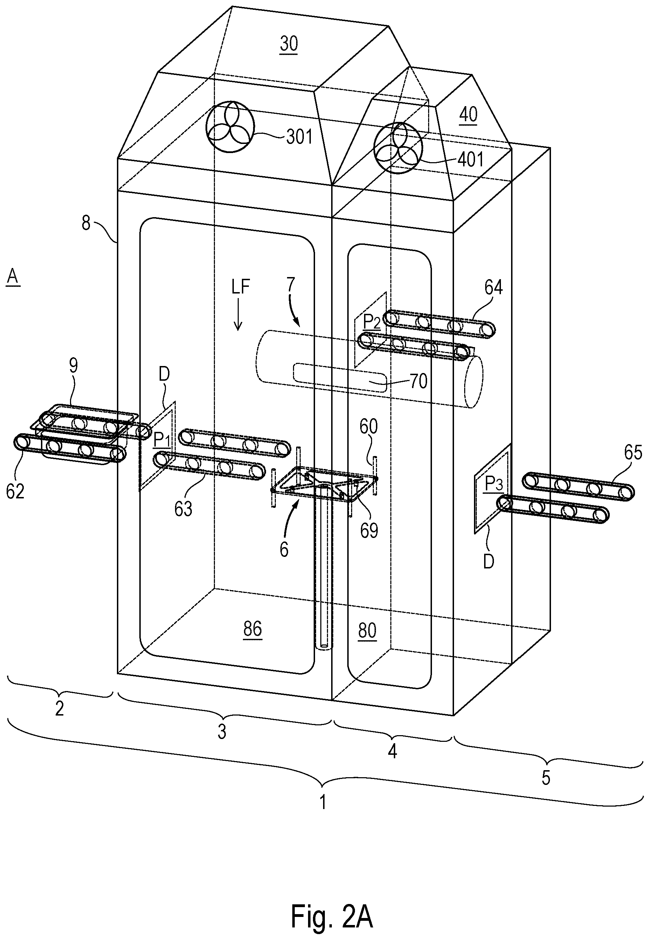

Figur 2A - Arbeitsphase 1 (Startsituation), in transparenter Darstellung;

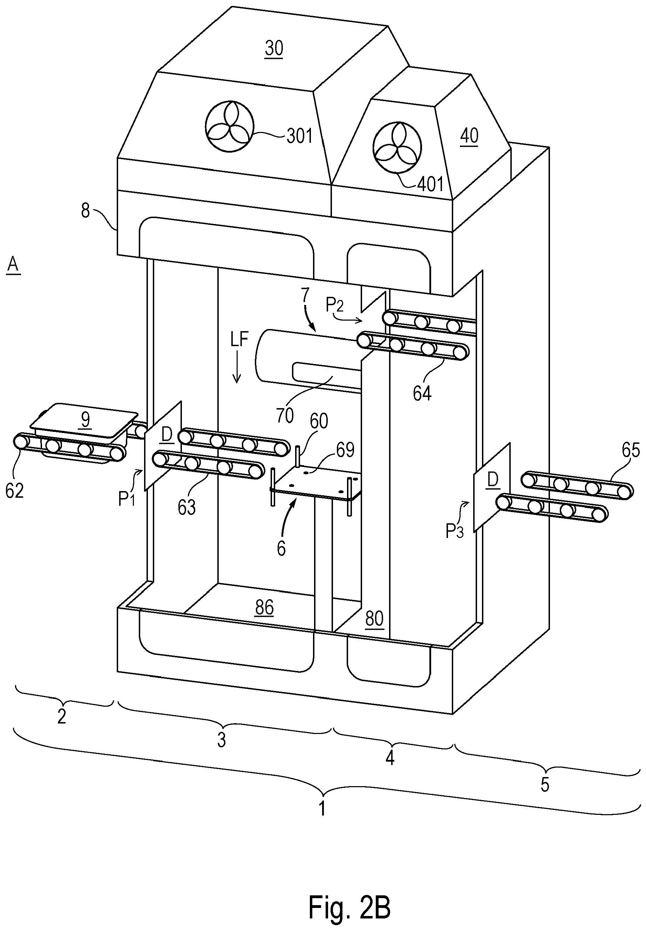

Figur 2B - Arbeitsphase 1 (Startsituation), im Teilschnitt;

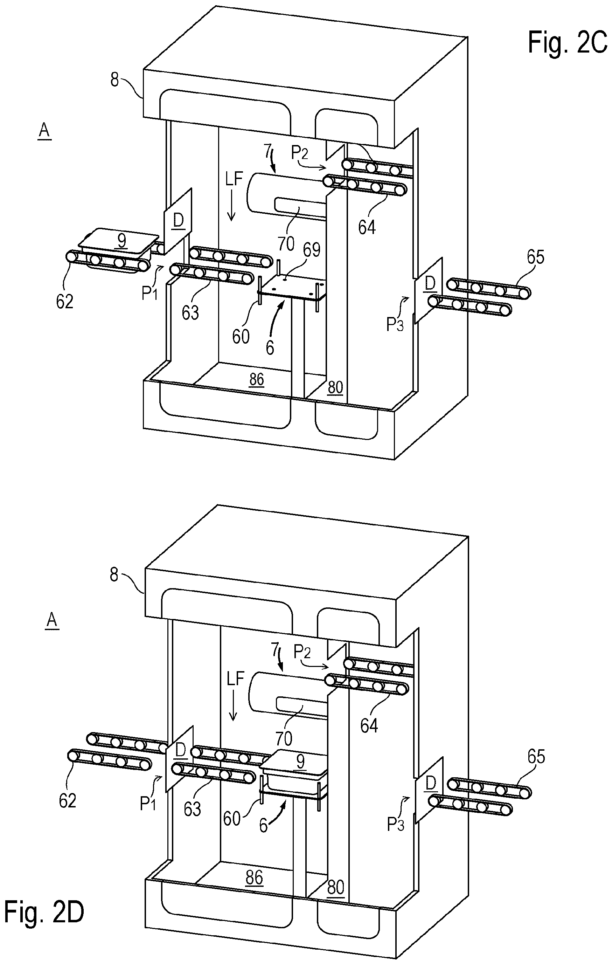

Figur 2C - Arbeitsphase 2;
Figur 2D - Arbeitsphase 3;

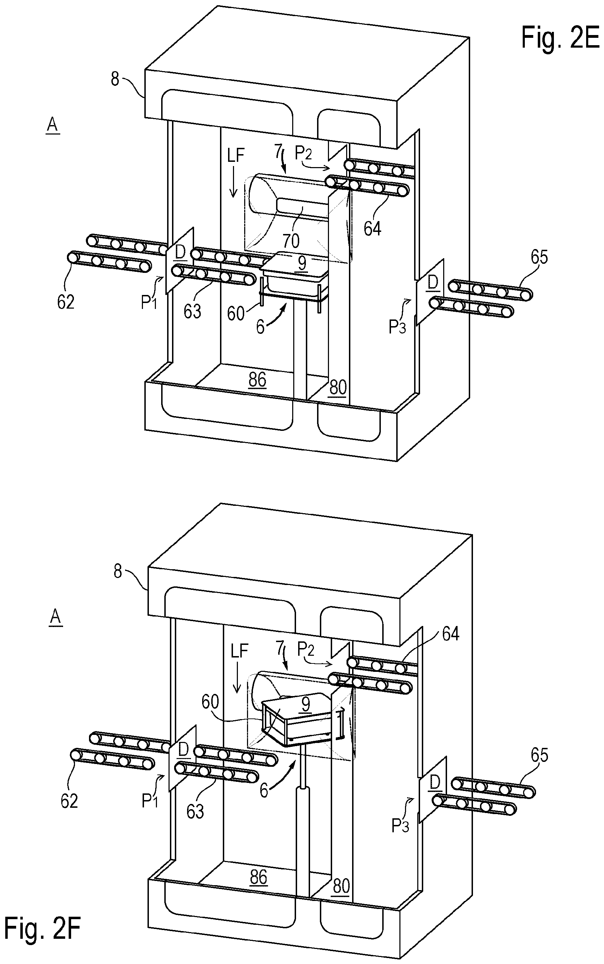

Figur 2E - Arbeitsphase 4;
Figur 2F - Arbeitsphase 5;

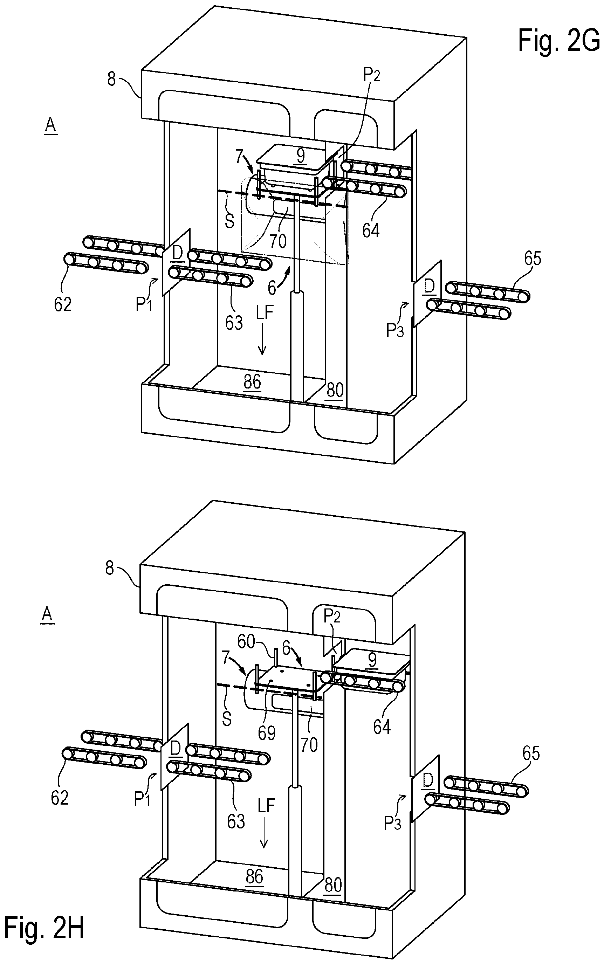

Figur 2G - Arbeitsphase 6;
Figur 2H - Arbeitsphase 7;

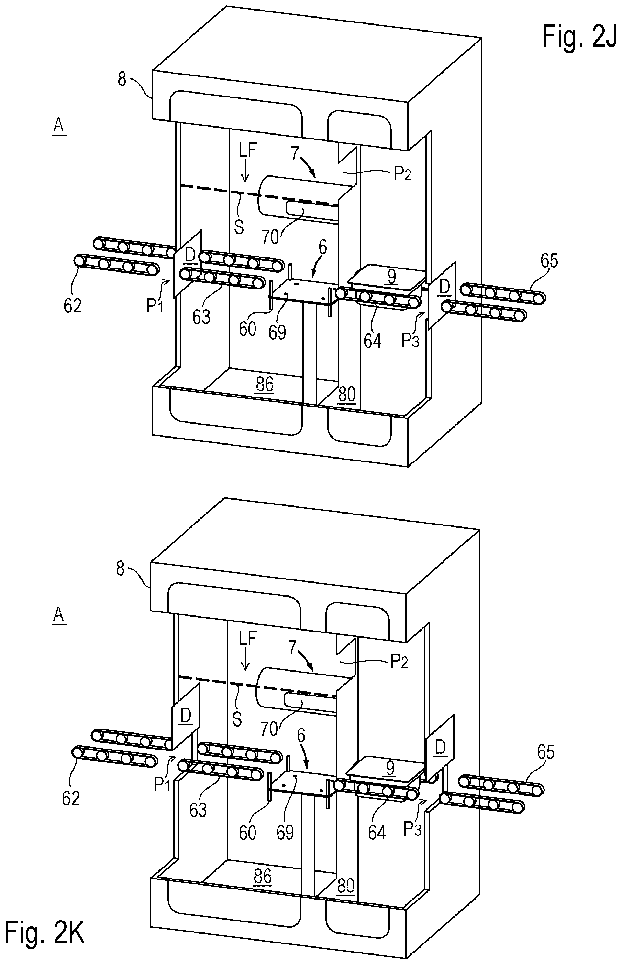

Figur 2J - Arbeitsphase 8;
Figur 2K - Arbeitsphase 9; und

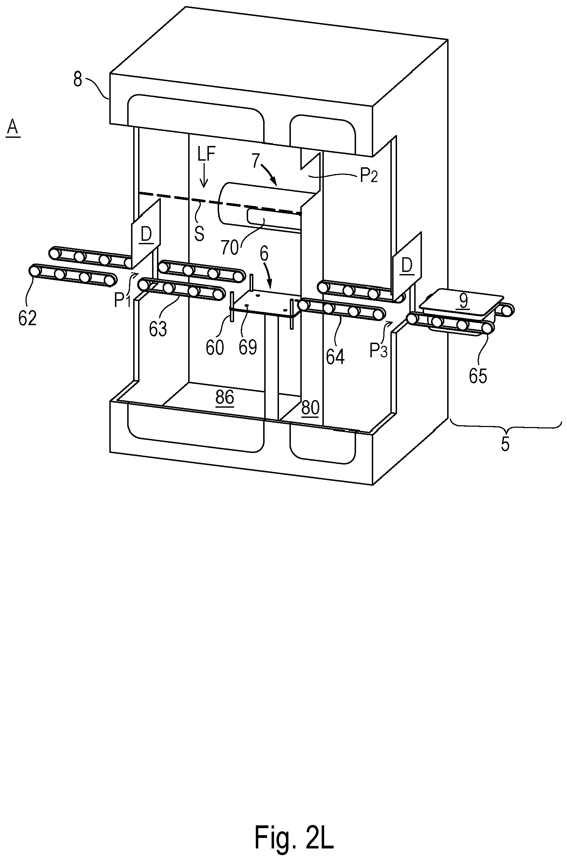

Figur 2L - Arbeitsphase 10

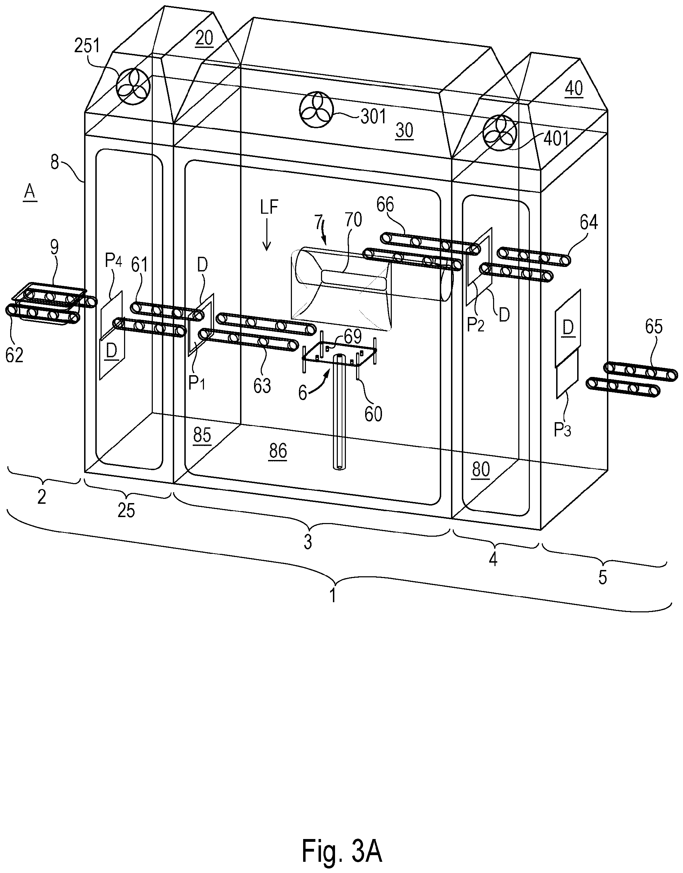

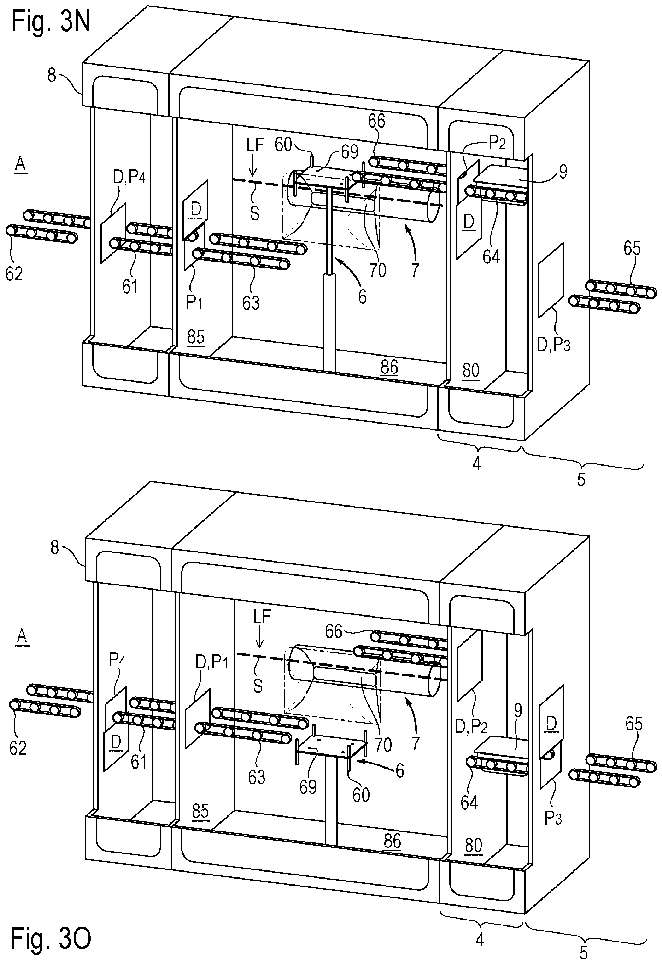

Figuren 3A bis 3P: die erfindungsgemässe Produktionsanlage in einer *zweiten Ausführungsform,* ergänzt mit einer der Prozesskammer vorgesetzten Eingangsschleuse, wiederum mit auf- und abwärts führendem Bewegungsverlauf während eines vollständigen Produktionszyklus;
Figur 3A - Arbeitsphase 1 (Startsituation), in transparenter Darstellung;

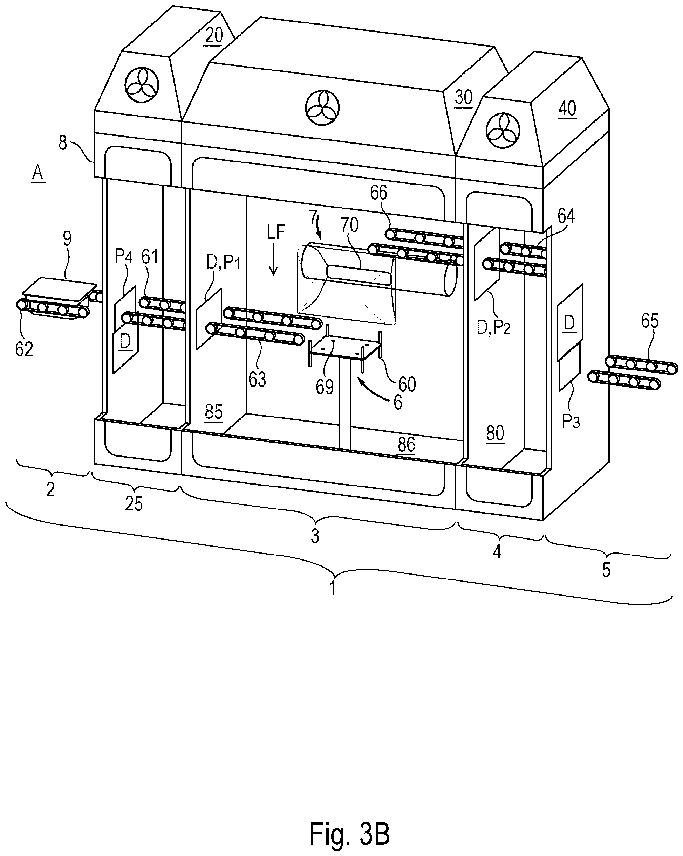

Figur 3B - Arbeitsphase 1 (Startsituation), im Teilschnitt;

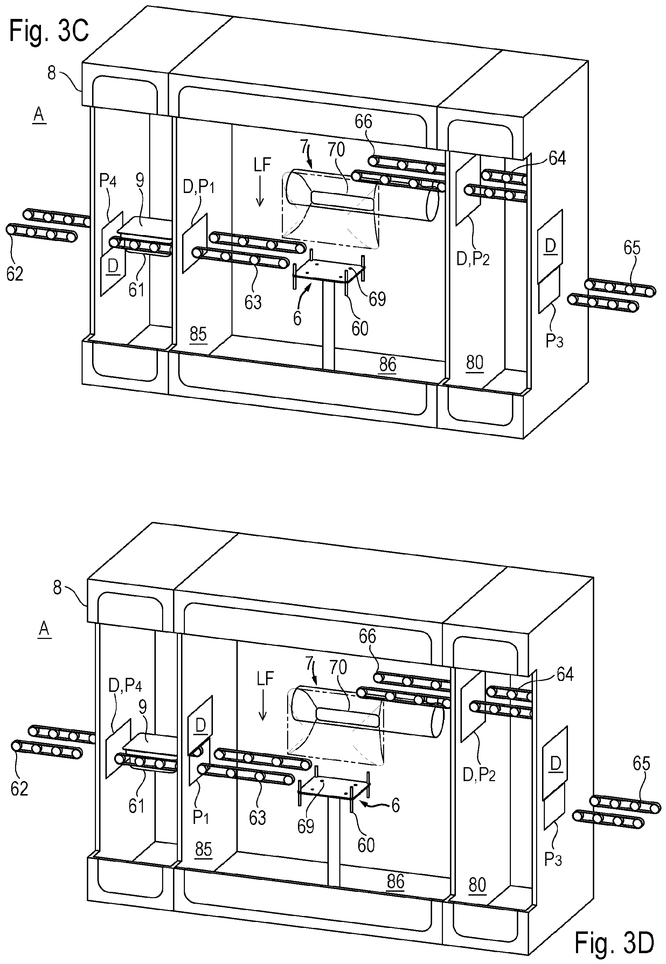

Figur 3C - Arbeitsphase 2;
Figur 3D - Arbeitsphase 3;

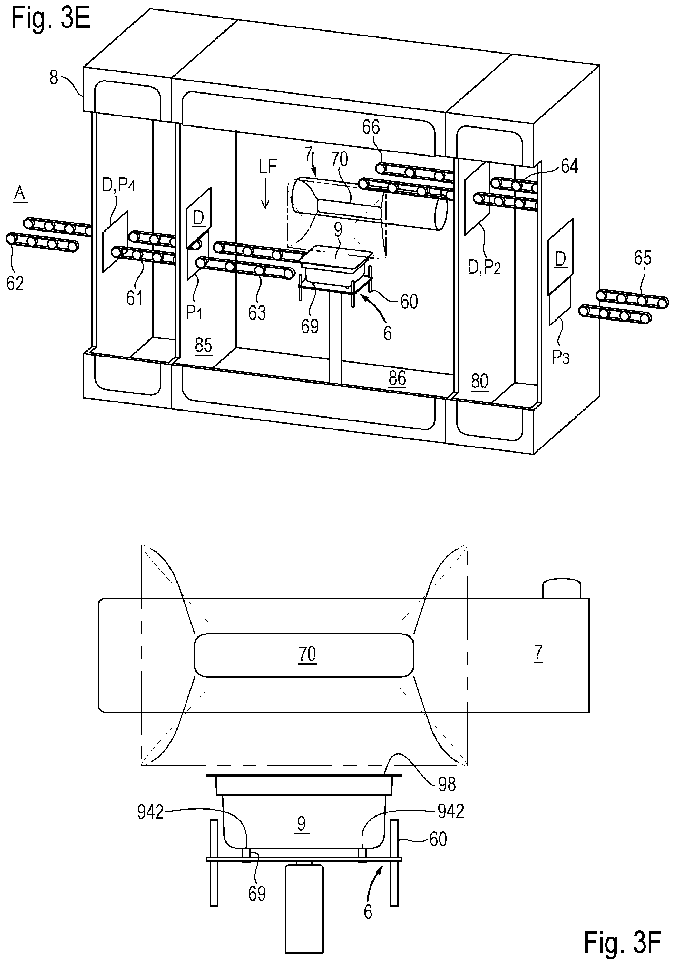

Figur 3E - Arbeitsphase 4;
Figur 3F - eine Vergrösserung aus Figur 3E im Umkreis von Elektronen-Strahlungsquelle und Gebinde;

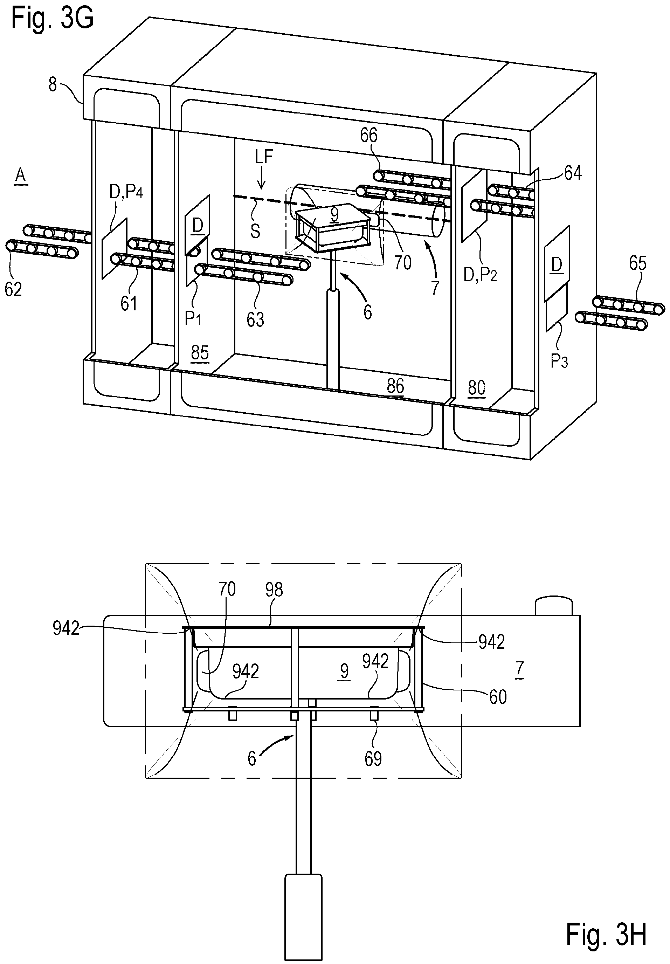

Figur 3G - Arbeitsphase 5;
Figur 3H - eine Vergrösserung aus Figur 3G im Umkreis von Elektronen-Strahlungsquelle und Gebinde;

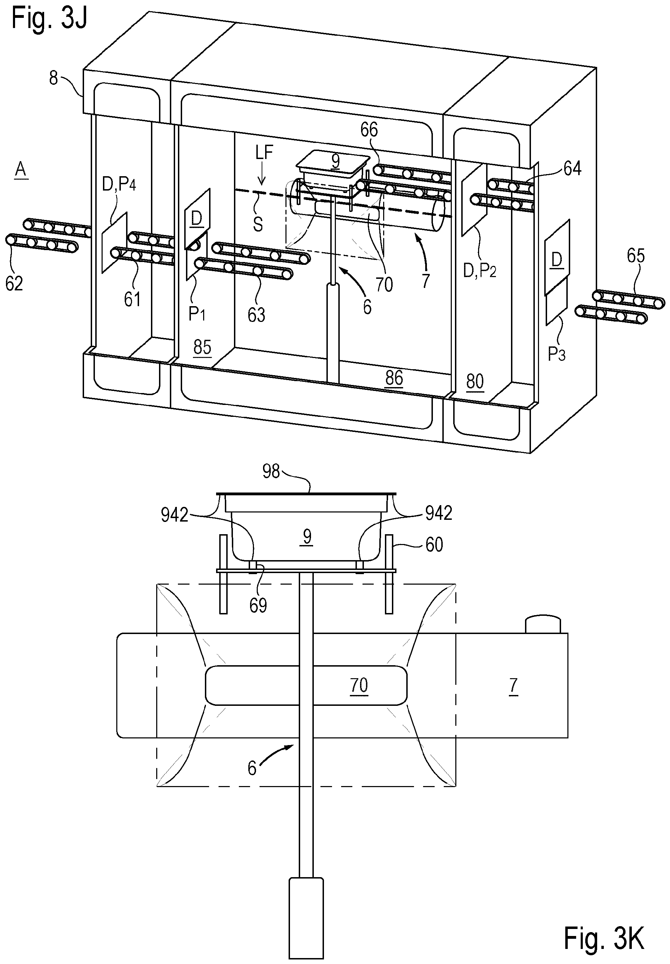

Figur 3J - Arbeitsphase 6;
Figur 3K - eine Vergrösserung aus Figur 3J im Umkreis von Elektronen-Strahlungsquelle und Gebinde;

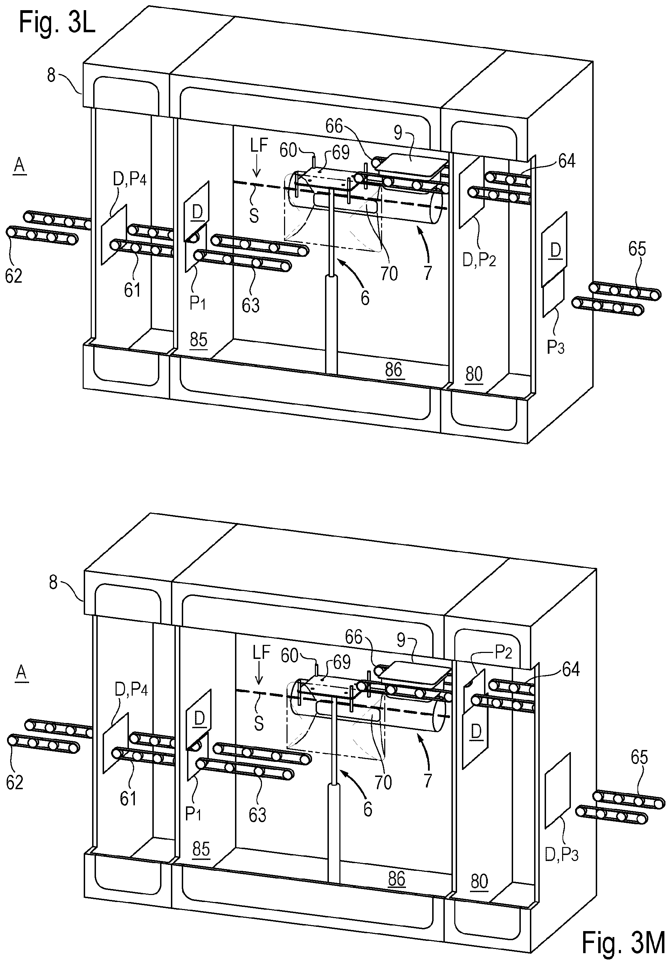

Figur 3L - Arbeitsphase 7;
Figur 3M - Arbeitsphase 8;
Figur 3N - Arbeitsphase 9;
Figur 30 - Arbeitsphase 10; und

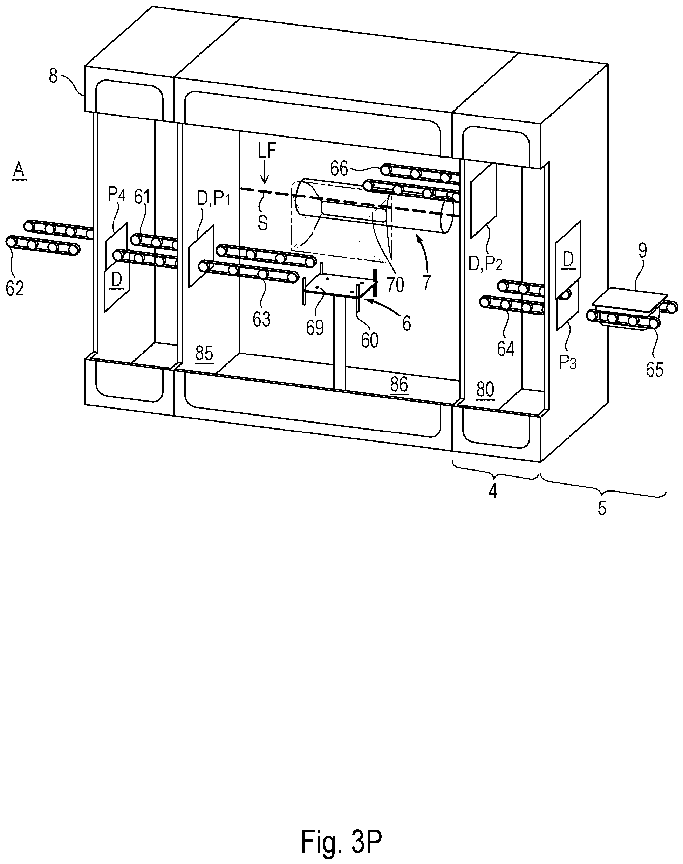

Figur 3P - Arbeitsphase 11

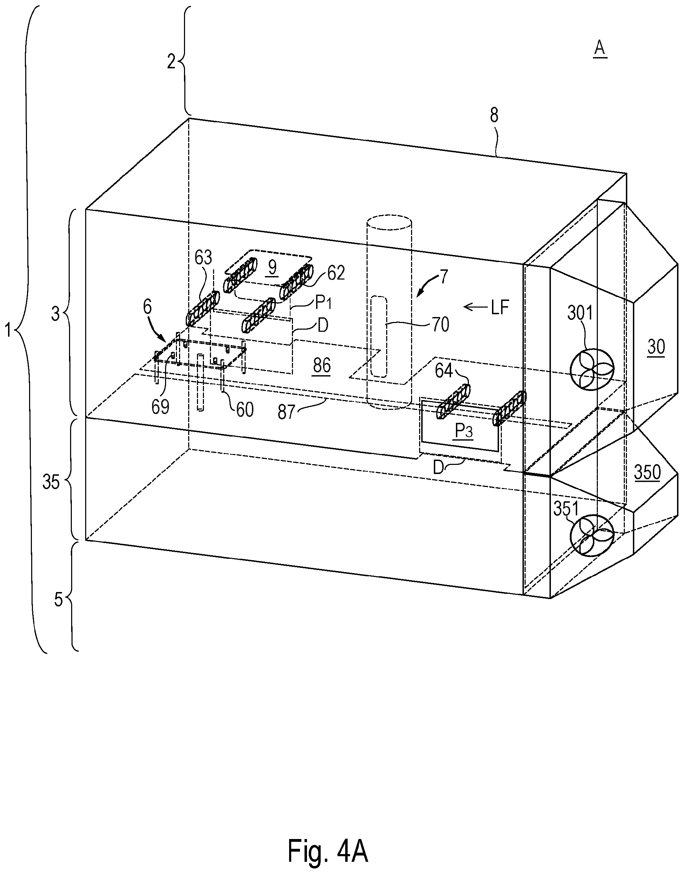

Figuren 4A bis 4N: die erfindungsgemässe Produktionsanlage in einer *dritten Ausführungsform,* mit im Prinzip in der Horizontalen durchgeführtem Bewegungsverlauf während eines vollständigen Produktionszyklus;
Figur 4A - Arbeitsphase 1 (Startsituation), in transparenter Darstellung;

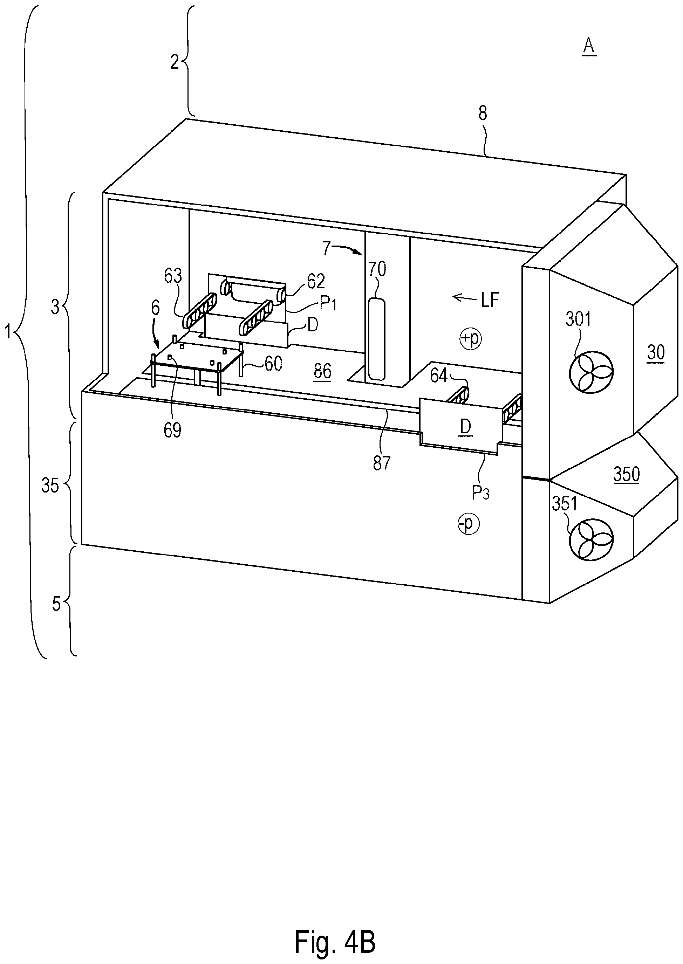

Figur 4B - Arbeitsphase 1 (Startsituation), im Teilschnitt;

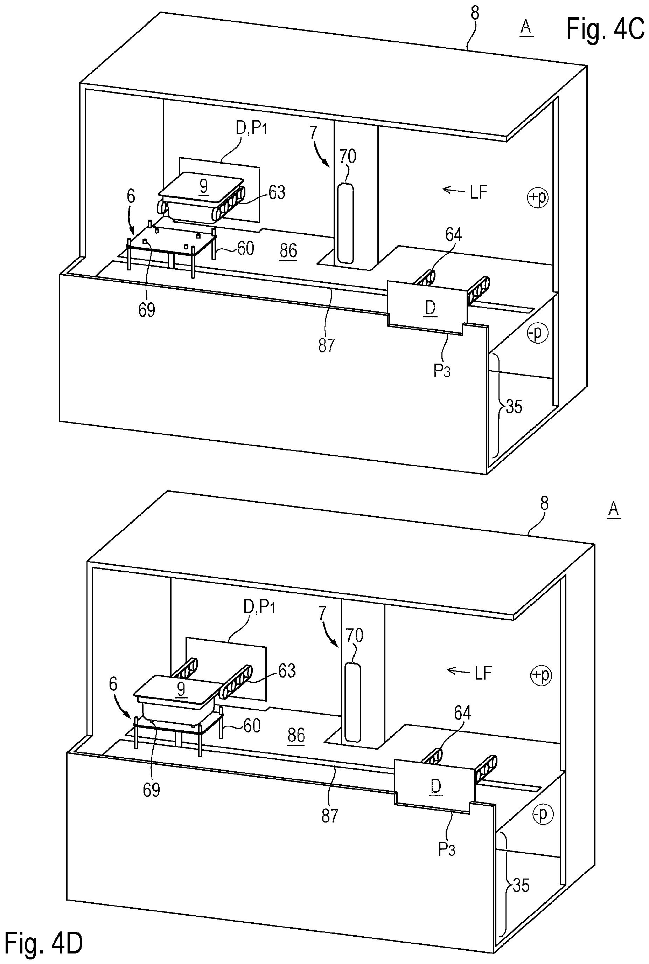

Figur 4C - Arbeitsphase 2;
Figur 4D - Arbeitsphase 3;

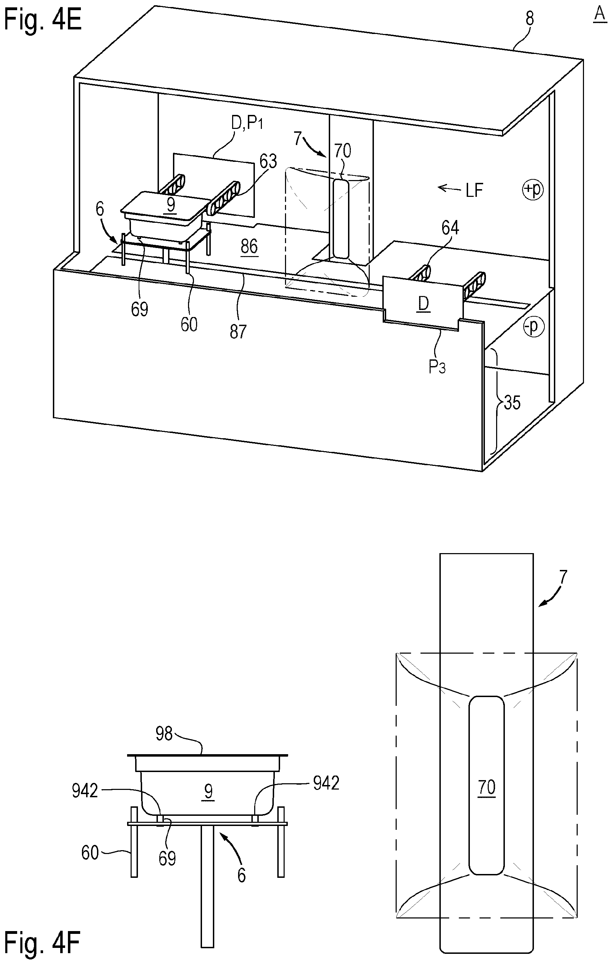

Figur 4E - Arbeitsphase 4;
Figur 4F - eine Vergrösserung aus Figur 4E im Umkreis von Elektronen-Strahlungsquelle und Gebinde;

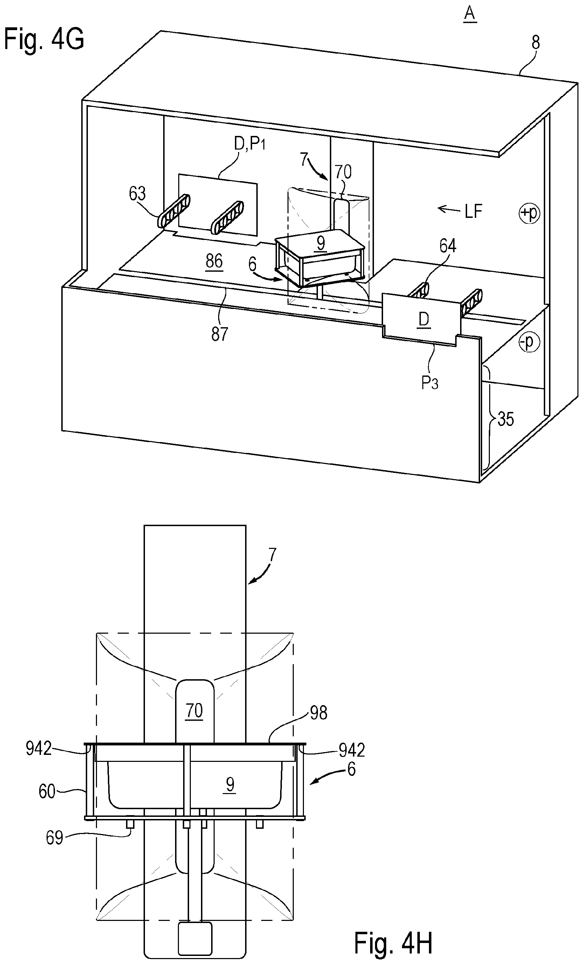

Figur 4G - Arbeitsphase 5;
Figur 4H - eine Vergrösserung aus Figur 4G im Umkreis von Elektronen-Strahlungsquelle und Gebinde;

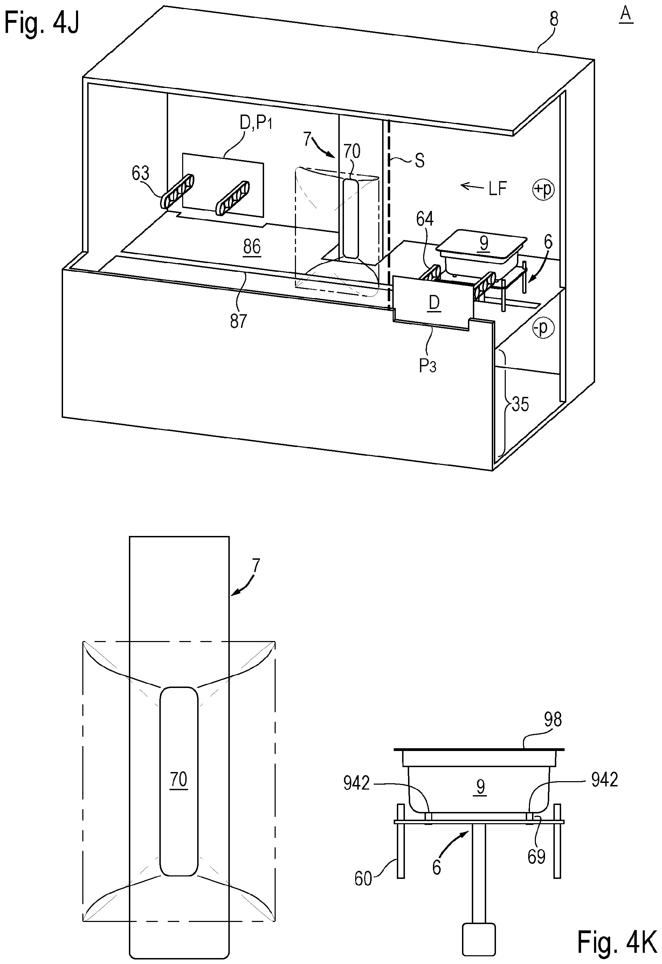

Figur 4J - Arbeitsphase 6;
Figur 4K - eine Vergrösserung aus Figur 4J im Umkreis von Elektronen-Strahlungsquelle und Gebinde;

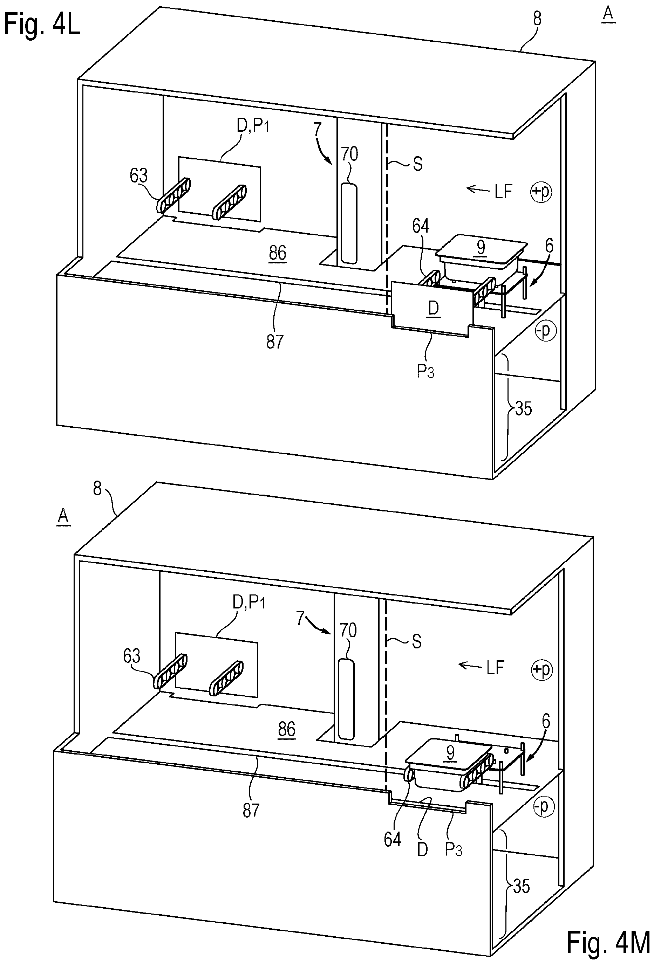

Figur 4L - Arbeitsphase 7;
Figur 4M - Arbeitsphase 8; und

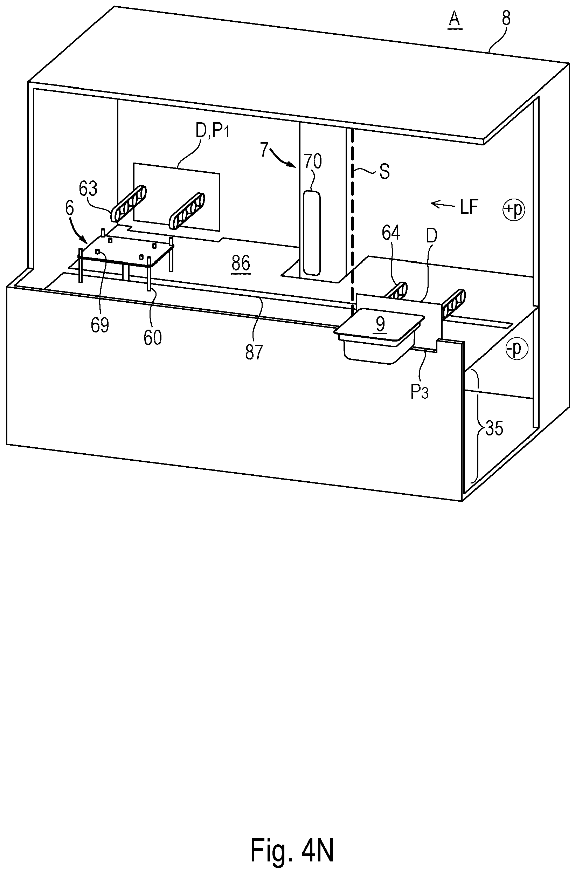

Figur 4N - Arbeitsphase 9

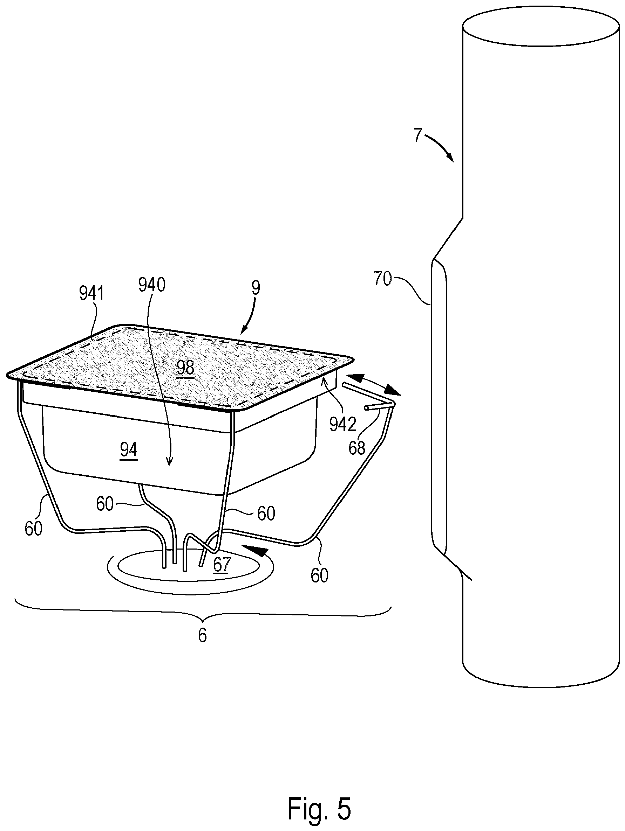

Figur 5 - eine alternative Gestaltung der Fördereinrichtung mit einzeln schaltbaren Stützelementen, positioniert vor dem Austrittsfenster der Elektronen-Strahlungsquelle;

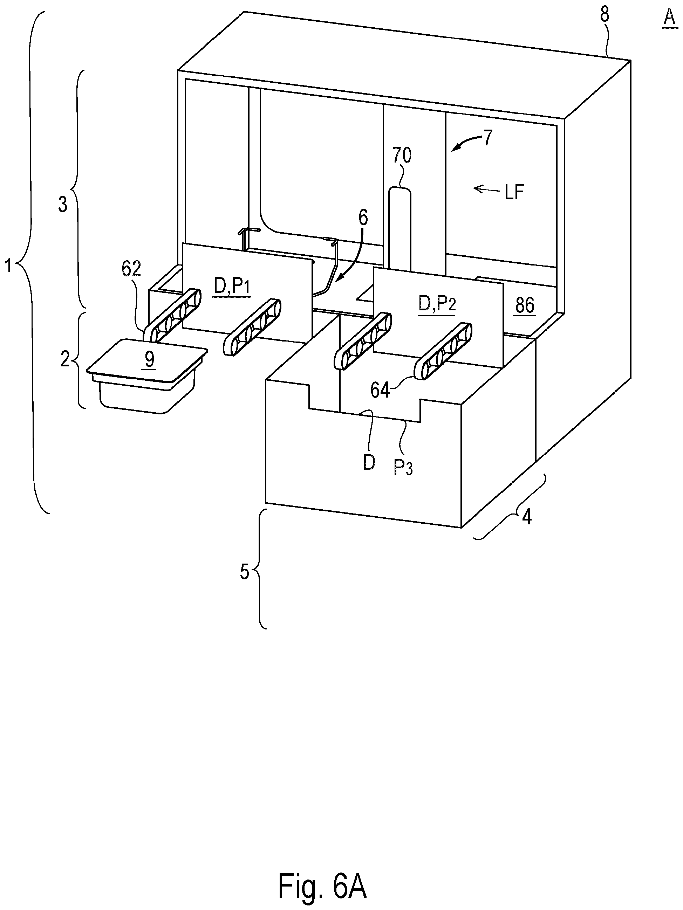

Figuren 6A bis 6J: die erfindungsgemässe Produktionsanlage in einer *vierten Ausführungsform,* mit Verwendung der Fördereinrichtung gemäss Figur 5 und im Prinzip wiederum in der Horizontalen durchgeführten Bewegungsverlauf während eines vollständigen Produktionszyklus;
Figur 6A - Arbeitsphase 1 (Startsituation);

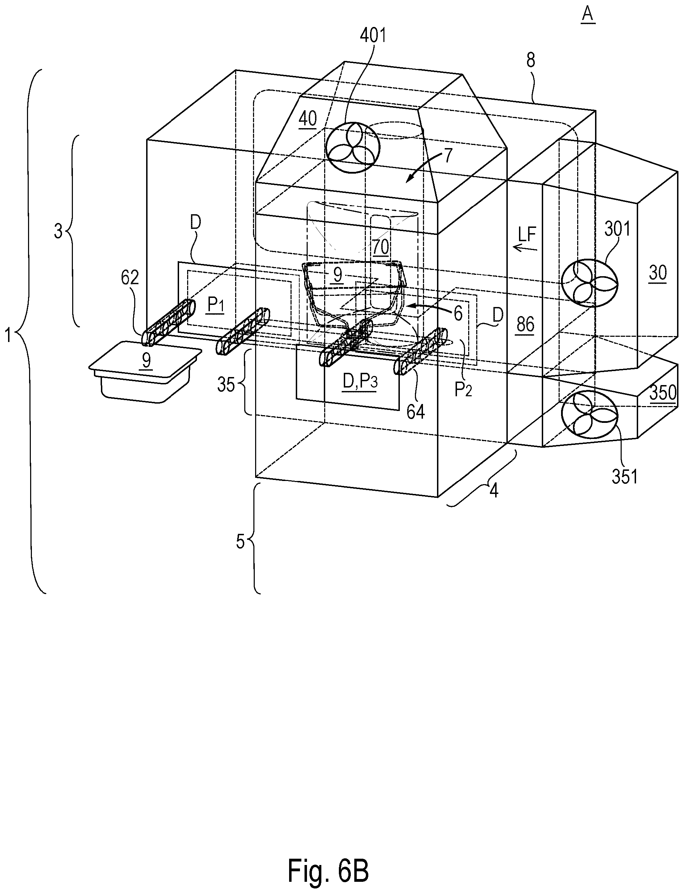

Figur 6B - Arbeitsphase X, in transparenter Darstellung;

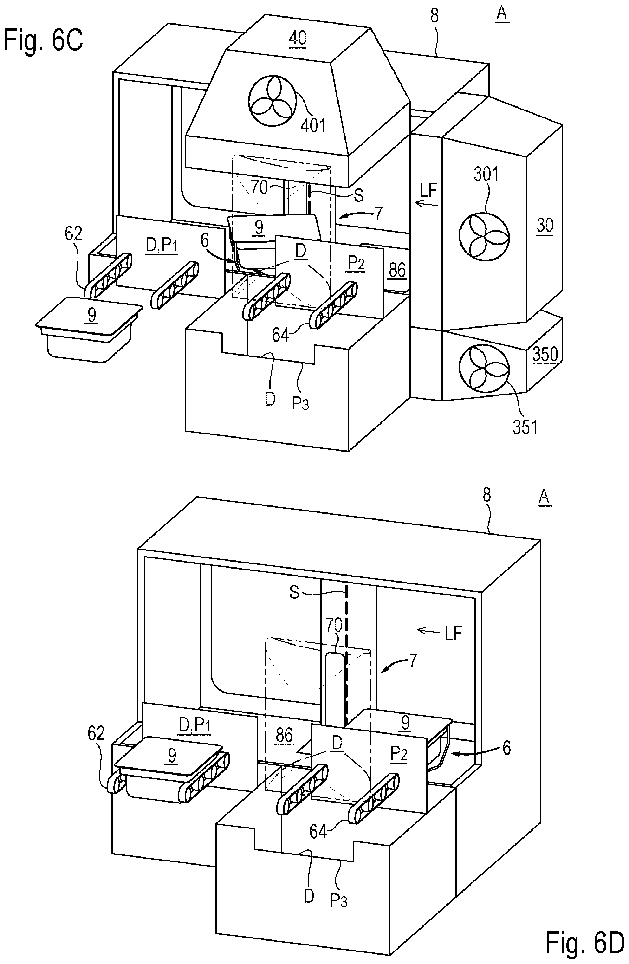

Figur 6C - Arbeitsphase X, im Teilschnitt;
Figur 6D - Arbeitsphase X+1;

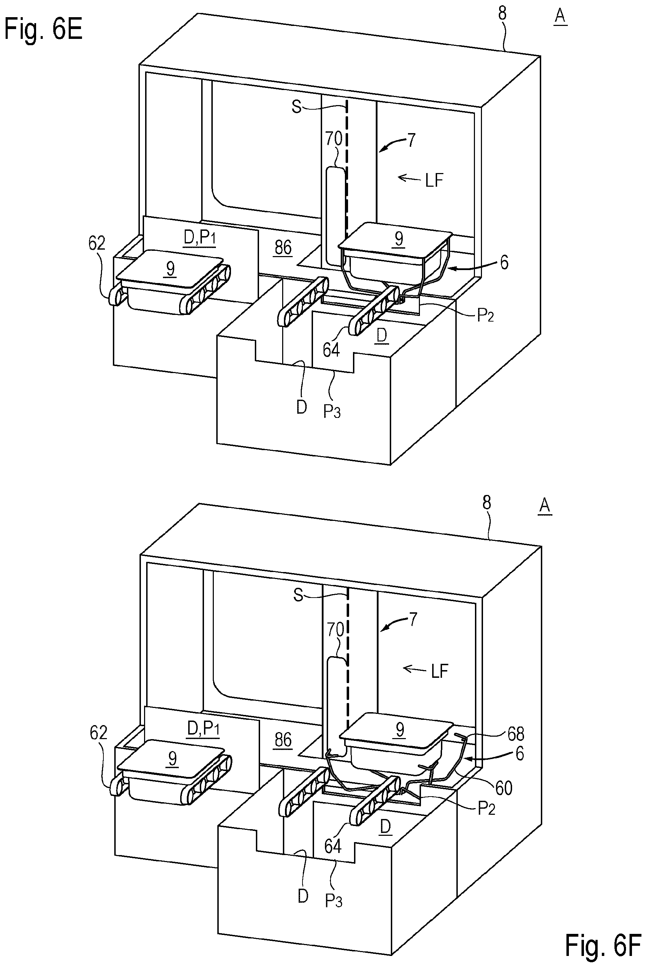

Figur 6E - Arbeitsphase X+2;
Figur 6F - Arbeitsphase X+3;

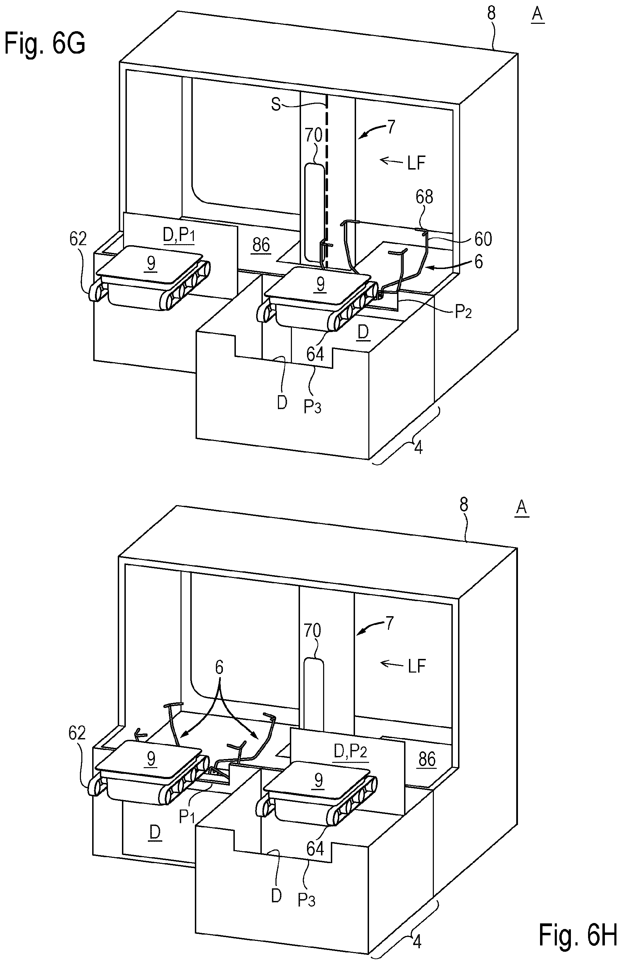

Figur 6G - Arbeitsphase X+4;
Figur 6H - Arbeitsphase X+5; und

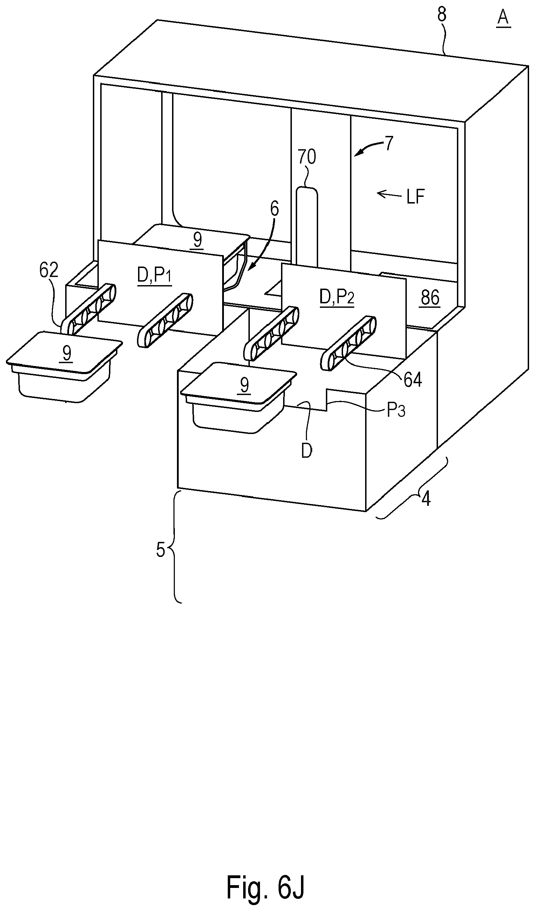

Figur 6J - Arbeitsphase X+6

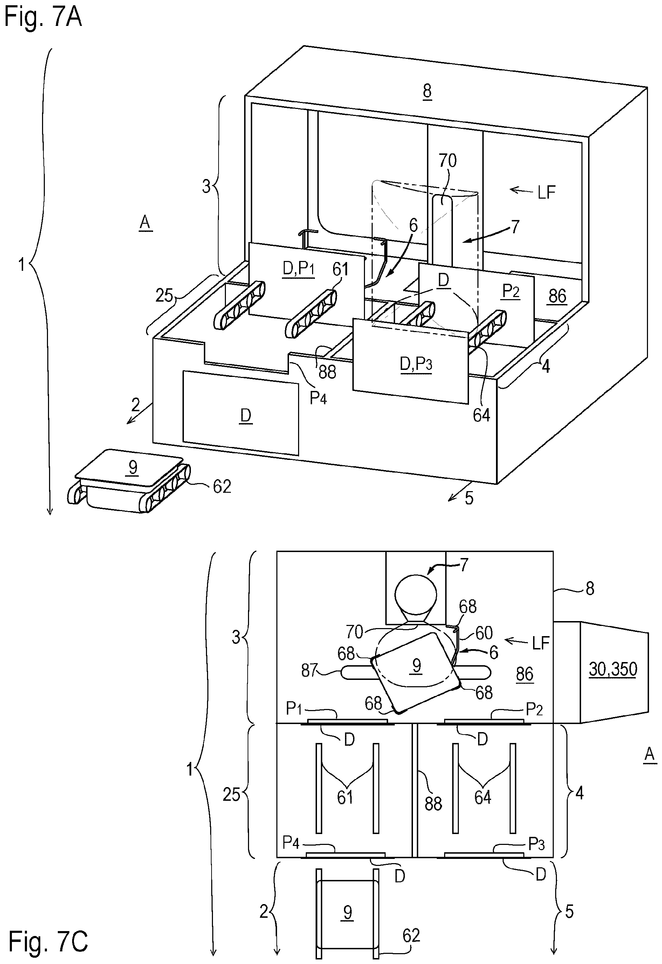

Figuren 7A bis 7K: die erfindungsgemässe Produktionsanlage in einer fünften Ausführungsform, ergänzt mit der Prozesskammer vorgesetzter Eingangsschleuse, und wiederum in der Horizontalen durchgeführten Bewegungsverlauf während eines vollständigen Produktionszyklus;
Figur 7A - Arbeitsphase 1 (Startsituation);

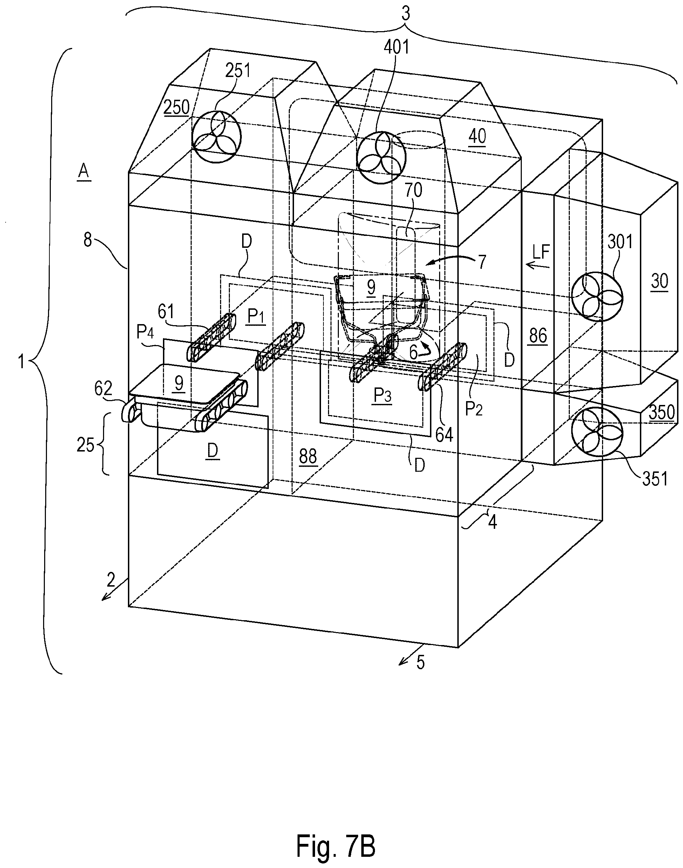

Figur 7B - Arbeitsphase X, in transparenter Darstellung;
Figur 7C - Arbeitsphase X, in prinzipieller Draufsicht;

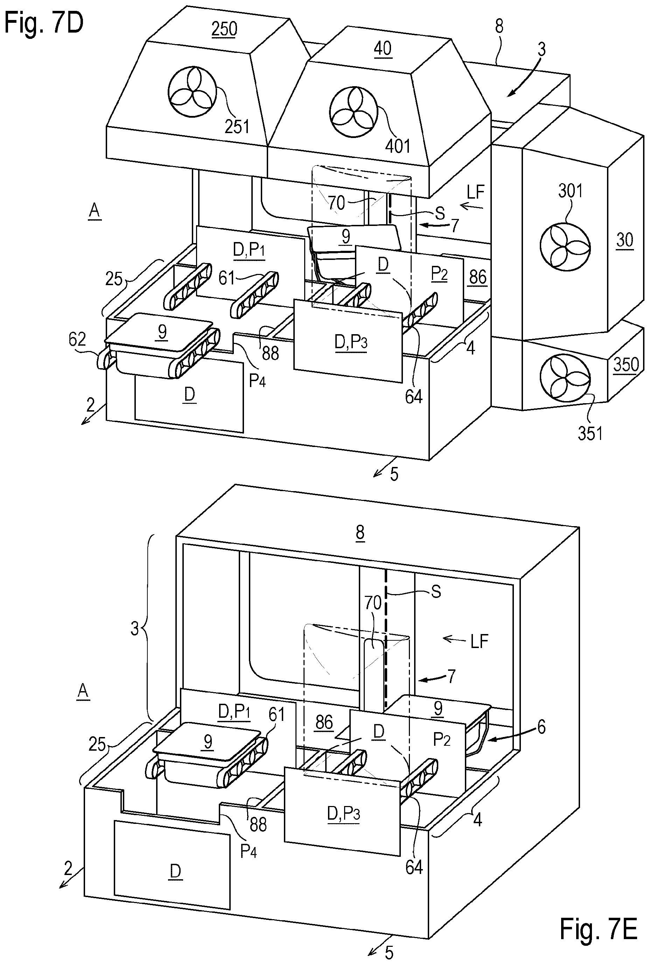

Figur 7D - Arbeitsphase X, im Teilschnitt;
Figur 7E - Arbeitsphase X+1;

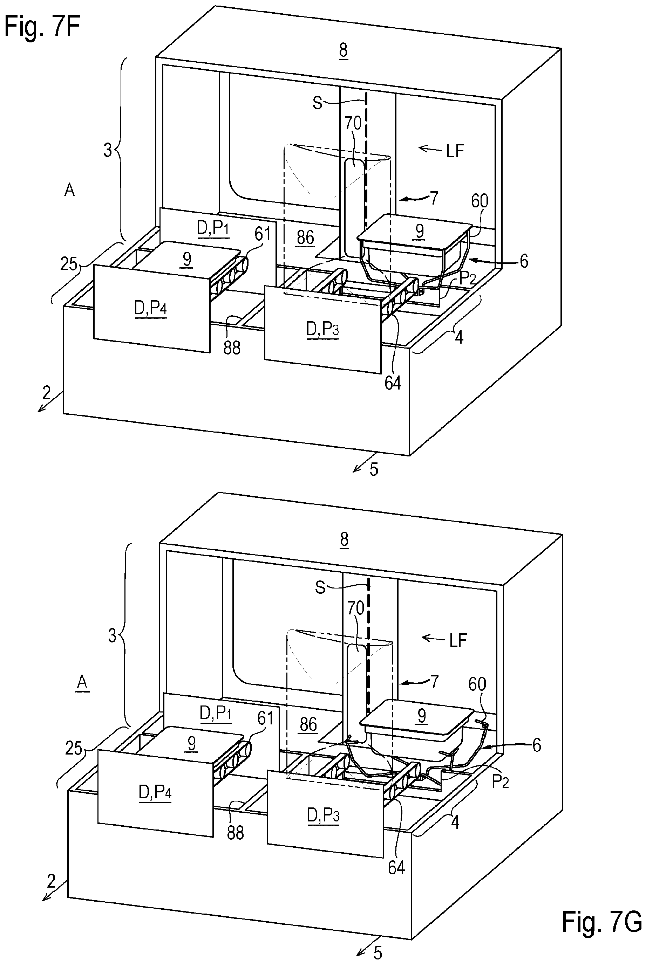

Figur 7F - Arbeitsphase X+2;
Figur 7G - Arbeitsphase X+3;

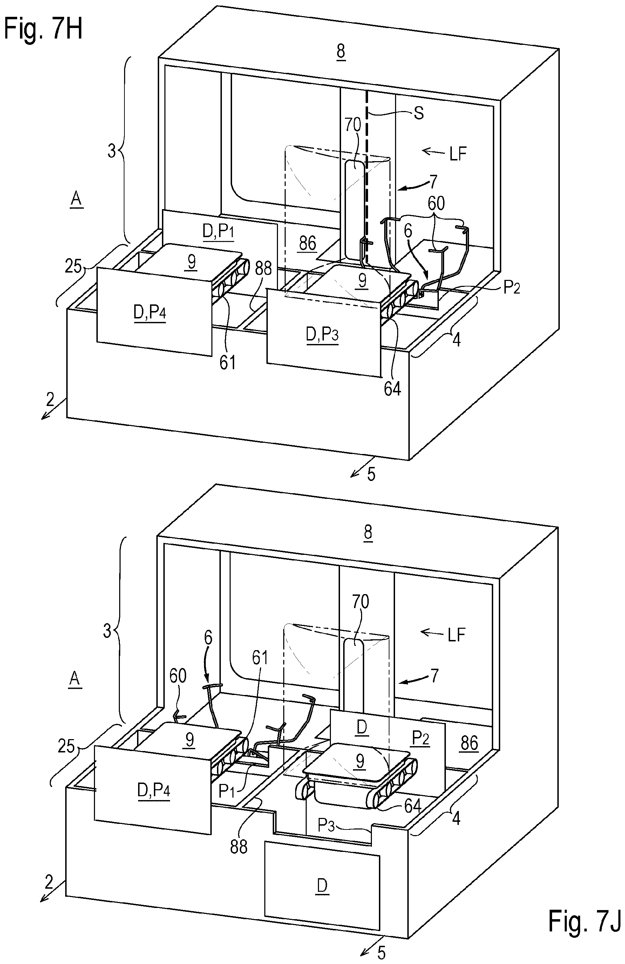

Figur 7H - Arbeitsphase X+4;
Figur 7J - Arbeitsphase X+5; und

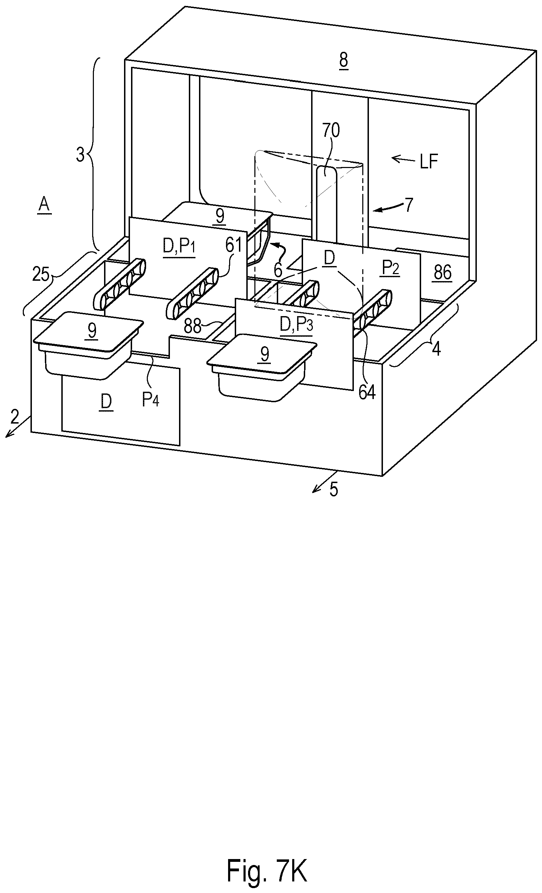

Figur 7K - Arbeitsphase X+6.

Mit Bezug auf die beiliegenden Zeichnungen erfolgt nachstehend die detaillierte Beschreibung des erfindungsgemässen Verfahrens mit dazu in Varianten konzipierter Produktionsanlage zum seriellen Transfer von Gebinden durch eine Prozesskammer zur Weiterverarbeitung in einem Containment unter aseptischen Bedingungen. Dies mittels einer in einer Prozesskammer installierten Elektronen-Strahlungsquelle, welche ein Austrittsfenster besitzt, aus dem ein Emissionsfeld von Elektronen austritt.

Für die gesamte weitere Beschreibung gilt folgende Festlegung. Sind in einer Figur zum Zweck zeichnerischer Eindeutigkeit Bezugsziffern enthalten und dabei zeichnerisch eindeutig erkennbar ist, dass es sich um "wiederkehrende" Bauteile handelt, aber im unmittelbar zugehörigen Beschreibungstext nicht erläutert, so sei im Interesse der Verkürzung, auf deren Erklärung in vorangehenden Figurenbeschreibungen hingewiesen.

### Figuren 1A und 1 B

Ein einzelnes Gebinde **9** umfasst:
- ein Behältnis **94** mit einem aseptischen Innenraum **940** und einem Durchlass **95,** der mit einer Abdeckung **98** verschlossen ist;
- ein im Innenraum **940** gelagertes Nest **96,** in dessen muldenförmigen Aufnahmekonturen die Artikel **960** stecken, welche nach Öffnen der Abdeckung **98** im Containment **5** zu behandeln sind;
- optional eine zwischen der Abdeckung **98** und dem Nest **96,** über den Artikeln **960** eingefügte Zwischenlage **97;** sowie
- eine im Anlieferungszustand das gesamte Gebinde **9** umgebende, geschlossene Umhüllung **99,** die das Innenvolumen **90** steril hält.

Das Behältnis **94** ist z.B. ein wannenförmiges Tub. Die Artikel **960** sind insbesondere Phiolen, Vials oder Spritzen. Von der Abdeckung **98** und der Umhüllung **99** bestehen vorzugsweise zumindest Flächenanteile aus einem semipermeablen Vliesgewebe, wie Tyvek^{®}. Die Abdeckung **98** ist typischerweise auf dem den Durchlass **95** des Behältnisses **94** umlaufenden Rand **941** versiegelt und bewahrt so den Innenraum **940** des Behältnisses **94** in sterilem Zustand. Das Behältnis **94** weist verschiedene Oberflächenareale **942** auf, die sich als Konturen zum Unterfassen oder klemmenden Ergreifen des Gebindes **9** eignen.

### Figuren 2A bis 2L

Die Produktionsanlage **1** - hier gezeigt in einer *ersten Ausführungsform* - mit aufwärts und abwärts führendem Bewegungsverlauf der zu behandelnden Gebinde **9** steht in einem Aufstellungsraum **A** mit definierter Reinraumklasse und umfasst die Prozesskammer **3,** welcher eine in den Aufstellraum **A** erstreckende Bereitstellungsstation **2** vorgesetzt ist. An die Prozesskammer **3** schliesst eine Abgangskammer **4** an, wobei beide durch die erste Trennwand **80** voneinander separiert sind und es von der Abgangskammer **4** in ein nicht dargestelltes Containment **5** übergeht. Unten schliesst die Prozesskammer **3** mit dem Boden **86** ab und über ihr ist das Plenum **30** mit dem darin eingebauten Ventilator **301** angeordnet. Äquivalent dazu befindet sich über der Abgangskammer **4** das Plenum **40** mit dem darinsitzenden Ventilator **401.** Prozesskammer **3** und Abgangskammer **4** sind vom Gehäuse **8** umgeben.

In der Bereitstellungsstation **2** sind zum Transport der Gebinde **9** ein erstes Transfermittel **62,** in der Prozesskammer **3** ein zweites Transfermittel **63,** in der Abgangskammer **4** ein drittes Transfermittel **64** und hin zum Containment **5** ein viertes Transfermittel **65** positioniert. In der Prozesskammer **3** ist die Fördereinrichtung **6** installiert, welche dem Transport der zu behandelnden Gebinde **9** durch die aus dem Austrittsfenster **70** der ebenfalls in der Prozesskammer **3** angeordneten Elektronen-Strahlungsquelle **7** emittierten Elektronenwolke dient. An der Fördereinrichtung **6** sind erste Stützelemente **60** und schaltbare zweite Stützelemente **69** zum Tragen der Gebinde **9** an verschiedenen Oberflächenarealen **942** vorgesehen.

Von der Bereitstellungsstation **2** in die Prozesskammer **3** führt eine erste im Gehäuse **8** angebrachte Passage **P1,** die sich mit einem beweglichen Torelement **D** schliessen bzw. öffnen lässt. Von der Prozesskammer **3** in die Abgangskammer **4** führt eine zweite in der ersten Trennwand **80** angebrachte Passage **P2;** und von der Abgangskammer **4** hin zum Containment **5** führt eine dritte im Gehäuse **8** angebrachte Passage **P3,** die sich ebenfalls mit einem beweglichen Torelement **D** schliessen bzw. öffnen lässt.

### Figuren 2A und 2B-Arbeitsphase 1 (Startsituation)

- In Prozesskammer **3** und Abgangskammer **4** herrschen nach einer Initial-Dekontamination - durchgeführt z.B. mit der Vorrichtung gemäss der WO 2019/165 564 A1 - sterile Bedingungen;
- durch die Prozesskammer **3** fliesst eine gleichgerichtete Verdrängungsströmung **LF,** welche hier von oben in die Prozesskammer **3** eingebracht und unten und/oder seitlich aus der Prozesskammer **3** abgeführt wird;
- erstes Transfermittel **62** in der Bereitstellungsstation **2** ist mit einem zur Dekontaminationsbehandlung vorgesehenem Gebinde **9** beladen;
- erste Passage **P1** und dritte Passage **P3** sind vom jeweils zugehörigen Torelement **D** verschlossen;
- zweiter Passage **P2** ist kein Torelement **D** zugehörig und diese steht daher offen;
- zweite, dritte und vierte Transfermittel **63,64,65** sind unbelegt;
- Fördereinrichtung **6** steht in abgesenkter Ruheposition, hierbei sind deren zweite Stützelemente **69** inaktiv, eingezogen geschaltet; und
- Elektronen-Strahlungsquelle **7** ist abgeschaltet.

Bei der Beschreibung der sukzessiven Arbeitsphasen werden nach der Startsituation jeweils nur die Änderungen gegenüber der vorangehenden Arbeitsphase geschildert. Dies gilt auch für alle übrigen *Ausführungsformen* gemäss Figurenfolgen 3A-3P, 4A-4N, 6A-6J und 7A-7K.

### Figur 2C - Arbeitsphase 2

- Erste Passage **P1** ist während des Einbringens des Gebindes **9** in die Prozesskammer **3** offen, zugehöriges Torelement **D** ist weggeschwenkt; und
- Gebinde **9** wird durch erste Passage **P1** vom ersten Transfermittel **62** auf zweites Transfermittel **63** in der Prozesskammer **3** geladen.

### Figur 2D - Arbeitsphase 3

- Erste Passage **P1** ist vom zugehörigen Torelement **D** verschlossen; und
- Gebinde **9** wird vom zweiten Transfermittel **63** auf die Fördereinrichtung **6** geladen; hierbei unterfassen die zweiten Stützelemente **69** Oberflächenareale **942** am Gebinde **9.**

### Figur 2E - Arbeitsphase 4

- Elektronen-Strahlungsquelle **7** wird eingeschaltet.

### Figur 2F - Arbeitsphase 5

- Fördereinrichtung **6** transportiert das Gebinde **9** in einer kombinierten Aufwärts- und Drehbewegung durch die aus dem Austrittsfenster **70** der Elektronen-Strahlungsquelle **7** emittierten Elektronenwolke; hierbei lösen sich die zweiten Stützelemente **69** von den bisher unterfassten Oberflächenarealen **942** und die ersten Stützelemente **60** tragen das Gebinde **9** an anderen Oberflächenarealen **942,** so dass freiliegend sämtliche Oberflächenareale **942** am Gebinde **9** von der Elektronenwolke bestrahlt werden.

### Figur 2G - Arbeitsphase 6

- Fördereinrichtung **6** trägt das Gebinde **9** bis zur zweiten Passage **P2,** an das dritte Transfermittel **64;**
- die zweiten Stützelemente **69** fahren wieder hoch, so dass diese das Gebinde **9** wieder tragen, während die ersten Stützelemente **60** sich vom Gebinde **9** lösen; und
- nach dem Transport des Gebindes **9** durch das Emissionsfeld der Elektronen-Strahlungsquelle **7** und bei fliessender Verdrängungsströmung **LF** ist in der Prozesskammer **3** eine Sterilgrenze **5** entstanden, ab der in weiterer Verarbeitungsrichtung des Gebindes **9** Reinraumbedingungen herrschen, z.B. die Reinraumklasse A gemäss DIN EN ISO 14644-1.

### Figur 2H - Arbeitsphase 7

- Elektronen-Strahlungsquelle **7** wird abgeschaltet; und
- Gebinde **9** wird von der Fördereinrichtung **6** durch die zweite Passage **P2** auf das dritte Transfermittel **64** in der Abgangskammer **4** geladen.

### Figur 2J - Arbeitsphase 8

- Gebinde **9** wird vom dritten Transfermittel **64** in einer Abwärtsbewegung an die dritte Passage **P3** herangeführt; und
- Fördereinrichtung **6** fährt abwärts in Ausgangsposition zurück.

### Figur 2K - Arbeitsphase 9

- Erste Passage **P1** und dritte Passage **P3** werden durch Wegschwenken des jeweils zugehörigen Torelements **D** geöffnet.

### Figur 2L - Arbeitsphase 10

- Gebinde **9** wird vom dritten Transfermittel **64** durch die dritte Passage **P3** auf das vierte Transfermittel **65** im Containment **5** geladen; und
- ein nächster Behandlungszyklus kann mit der Arbeitsphase 1 gestartet werden.

### Figuren 3A bis 3P

Die Produktionsanlage **1** - hier gezeigt in einer erweiterten *zweiten Ausführungsform* - mit ebenfalls aufwärts und abwärts führendem Bewegungsverlauf der zu behandelnden Gebinde **9.** Für diese *zweite Ausführungsform* gelten die sich wiederholenden Merkmale der *ersten Ausführungsform* gemäss Figuren 2A-2L, ansonsten die nachstehend beschriebenen Abwandlungen.

Zwischen der Prozesskammer **3** und der Bereitstellungsstation **2** ist eine kammerförmige Eingangsschleuse **25** mit dem Plenum **20** und dem darin sitzenden Ventilator **251** angeordnet, wobei eine zweite Trennwand **85** die Prozesskammer **3** von der Eingangsschleuse **25** abgrenzt. In der zur Bereitstellungsstation **2** weisenden Wandung als Bestandteil des Gehäuses **8** befindet sich eine vierte Passage **P4** mit zugehörigem Torelement **D,** und die zweite Passage **P2** in der ersten Trennwand **80** zwischen der Prozesskammer **3** und der Abgangskammer **4** ist mit einem zugehörigem Torelement **D** versehen. In der Eingangsschleuse **25** befindet sich ein fünftes Transfermittel **61** und ein sechstes Transfermittel **66** ergänzt das in der Prozesskammer **3** bereits vorhandene zweiten Transfermittel **63.** Ein wesentlicher Unterschied besteht noch darin, dass der Strahlenschutz hier durch den Aufbau dieser Produktionsanlage **1** realisiert wird und somit die Elektronen-Strahlungsquelle **7** permanent eingeschaltet bleiben kann.

### Figuren 3A und 3B - Arbeitsphase 1 (Startsituation)

- In Prozesskammer **3** und Abgangskammer **4** herrschen nach einer Initial-Dekontamination sterile Bedingungen; die Initial-Dekontamination erstreckt sich nicht bis in die Eingangsschleuse **25;**
- durch die Prozesskammer **3** fliesst eine gleichgerichtete Verdrängungsströmung **LF,** welche hier ebenfalls von oben in die Prozesskammer **3** eingebracht und unten und/oder seitlich aus der Prozesskammer **3** abgeführt wird;
- erstes Transfermittel **62** in der Bereitstellungsstation **2** ist mit einem zur Dekontaminationsbehandlung vorgesehenem Gebinde **9** beladen;
- erste Passage **P1** und zweite Passage **P2** sind vom jeweils zugehörigen Torelement **D** verschlossen;
- dritte Passage **P3** und vierte Passage **P4** sind offen, jeweils zugehöriges Torelement **D** ist weggeschwenkt;
- fünfte, zweite, sechste, dritte und vierte Transfermittel **61,63,66,64,65** sind unbelegt;
- Fördereinrichtung **6** steht in abgesenkter Ruheposition, hierbei sind deren zweite Stützelemente **69** inaktiv, eingezogen geschaltet; und
- Elektronen-Strahlungsquelle **7** ist eingeschaltet.

### Figur 3C - Arbeitsphase 2

- Gebinde **9** wird durch vierte Passage **P4** vom ersten Transfermittel **62** auf fünftes Transfermittel **61** in der Eingangsschleuse **25** geladen.

### Figur 3D - Arbeitsphase 3

- Erste Passage **P1** wird geöffnet, zugehöriges Torelement **D** weggeschwenkt; und
- vierte Passage **P4** ist vom zugehörigen Torelement **D** verschlossen.

### Figuren 3E und 3F - Arbeitsphase 4

- Gebinde **9** wurde vom fünften Transfermittel **61** durch erste Passage **P1** in die Prozesskammer **3** zunächst auf zweites Transfermittel **63** und von dort auf die Fördereinrichtung **6** geladen; hierbei unterfassen die zweiten Stützelemente **69** Oberflächenareale **942** am Gebinde **9.**

### Figuren 3G und 3H - Arbeitsphase 5

- Fördereinrichtung **6** transportiert das Gebinde **9** in einer kombinierten Aufwärts- und Drehbewegung durch die aus dem Austrittsfenster **70** der Elektronen-Strahlungsquelle **7** emittierten Elektronenwolke; hierbei lösen sich die zweiten Stützelemente **69** von den bisher unterfassten Oberflächenarealen **942** und die ersten Stützelemente **60** tragen das Gebinde **9** an anderen Oberflächenarealen **942,** so dass freiliegend sämtliche Oberflächenareale **942** am Gebinde **9** von der Elektronenwolke bestrahlt werden; und
- nach dem Transport des Gebindes **9** durch das Emissionsfeld der Elektronen-Strahlungsquelle **7** und bei fliessender Verdrängungsströmung **LF** ist in der Prozesskammer **3** die Sterilgrenze **5** entstanden, ab der in weiterer Verarbeitungsrichtung des Gebindes **9** Reinraumbedingungen herrschen, z.B. die Reinraumklasse A gemäss DIN EN ISO 14644-1.

### Figuren 3J und 3K - Arbeitsphase 6

- Fördereinrichtung **6** trägt das Gebinde **9** bis an das sechste Transfermittel **66;** und
- die zweiten Stützelemente **69** fahren wieder hoch, so dass diese das Gebinde **9** wieder tragen, während die ersten Stützelemente **60** sich vom Gebinde **9** lösen.

### Figuren 3L - Arbeitsphase 7

- Gebinde **9** wird von der Fördereinrichtung **6** auf sechstes Transfermittel **66** geladen.

### Figuren 3M - Arbeitsphase 8

- Zweite Passage **P2** wird geöffnet, zugehöriges Torelement **D** weggeschwenkt; und
- dritte Passage **P3** wird vom zugehörigen Torelement **D** verschlossen.

### Figuren 3N - Arbeitsphase 9

- Gebinde **9** wird vom sechsten Transfermittel **66** durch zweite Passage **P2** auf drittes Transfermittel **64** in der Abgangskammer **4** geladen.

### Figuren 30 - Arbeitsphase 10

- Erste Passage **P1** und zweite Passage **P2** sind vom jeweils zugehörigen Torelement **D** verschlossen;
- dritte Passage **P3** und vierte Passage **P4** sind offen, jeweils zugehöriges Torelement **D** ist weggeschwenkt; und
- Gebinde **9** wird auf drittem Transfermittel **64** abwärts fahrend an dritte Passage **P3** herangeführt.

### Figuren 3P - Arbeitsphase 11

- Gebinde **9** wird vom dritten Transfermittel **64** durch dritte Passage **P3** auf viertes Transfermittel **65** im Containment **5** geladen; und
- ein nächster Behandlungszyklus kann mit der Arbeitsphase 1 gestartet werden.

### Figuren 4A bis 4N

Die Produktionsanlage **1** - nun aufgebaut in *dritter Ausführungsform* - mit im Prinzip in der Horizontalen durchgeführtem Bewegungsverlauf der zu behandelnden Gebinde **9.** Für diese *dritte Ausführungsform* gelten die sich wiederholenden Merkmale der *ersten Ausführungsform* gemäss Figuren 2A-2L, ansonsten die nachstehend beschriebenen Abwandlungen.

Die Bereitstellungsstation **2** befindet sich jetzt auf der Rückseite der Prozesskammer **3,** welche seitlich das Plenum **30** mit dem einblasenden Ventilator **301** hat, der eine laminare Luftströmung **LF** in die Prozesskammer **3** einbringt. Diese Luftströmung **LF** wird über den im Boden **86** vorgesehenen Luftauslass **87** abgeführt. Unterhalb der Prozesskammer **3** ist eine Basiskammer **35** angeordnet, welche seitlich das Plenum **350** mit dem absaugenden Ventilator **351** hat. Hierdurch entsteht in der Prozesskammer **3** ein Überdruck **+p** und in der Basiskammer **35** ein Unterdruck **-p.** Von der Vorderseite der Prozesskammer **3** geht es in das Containment **5** über.

### Figuren 4A und 4B - Arbeitsphase 1 (Startsituation)

- In Prozesskammer **3** herrschen nach einer Initial-Dekontamination - durchgeführt z.B. mit der Vorrichtung gemäss der WO 2019/165 564 A1 - sterile Bedingungen;
- durch die Prozesskammer **3** fliesst eine gleichgerichtete Verdrängungsströmung **LF,** welche hier von seitlich in die Prozesskammer **3** eingebracht und unten durch einen Luftauslass **87** aus der Prozesskammer **3** abgeführt wird;
- erstes Transfermittel **62** in der Bereitstellungsstation **2** ist mit einem zur Dekontaminationsbehandlung vorgesehenem Gebinde **9** beladen;
- erste Passage **P1** ist während des Einbringens des Gebindes **9** in die Prozesskammer **3** offen, zugehöriges Torelement **D** ist weggeschwenkt;
- dritte Passage **P3** als Übergang in das Containment **5** ist vom zugehörigen Torelement **D** verschlossen;
- zweites Transfermittel **63** steht unbelegt zur Übernahme des Gebindes **9** in der Prozesskammer **3** an erster Passage **P1** bereit;
- die bisher leere Fördereinrichtung **6** steht zur Übernahme des Gebindes **9** in der Prozesskammer **3** bereit; und
- Elektronen-Strahlungsquelle **7** ist abgeschaltet.

### Figur 4C - Arbeitsphase 2

- Erste Passage **P1** ist vom zugehörigen Torelement D verschlossen; und
- Gebinde **9** ist durch erste Passage **P1** vom ersten Transfermittel **62** auf zweites Transfermittel **63** in der Prozesskammer **3** geladen.

### Figur 4D - Arbeitsphase 3

- Gebinde **9** ist vom zweiten Transfermittel **63** auf die Fördereinrichtung **6** geladen; hierbei unterfassen die zweiten Stützelemente **69** Oberflächenareale **942** am Gebinde **9.**

### Figuren 4E und 4F Arbeitsphase 4

- Elektronen-Strahlungsquelle **7** wird eingeschaltet.

### Figuren 4G und 4H - Arbeitsphase 5

- Fördereinrichtung **6** transportiert das Gebinde **9** in einer kombinierten Seiten- und Drehbewegung durch die aus dem Austrittsfenster **70** der Elektronen-Strahlungsquelle **7** emittierten Elektronenwolke; hierbei lösen sich die zweiten Stützelemente **69** von den bisher unterfassten Oberflächenarealen **942** und die ersten Stützelemente **60** tragen das Gebinde **9** an anderen Oberflächenarealen **942,** so dass freiliegend sämtliche Oberflächenareale **942** am Gebinde **9** von der Elektronenwolke bestrahlt werden.

### Figuren 4J und 4K - Arbeitsphase 6

- Fördereinrichtung **6** trägt das Gebinde **9** bis zum dritten Transfermittel **64;** hierbei können die zweiten Stützelemente **69** wieder aktiviert werden, um für das Gebinde **9** die Trägerfunktion zu übernehmen; und
- nach dem Transport des Gebindes **9** durch das Emissionsfeld der Elektronen-Strahlungsquelle **7** und bei fliessender Verdrängungsströmung **LF** ist in der Prozesskammer **3** die Sterilgrenze **5** entstanden, ab der in weiterer Verarbeitungsrichtung des Gebindes **9** Reinraumbedingungen herrschen, z.B. die Reinraumklasse A gemäss DIN EN ISO 14644-1.

### Figur 4L - Arbeitsphase 7

- Elektronen-Strahlungsquelle **7** wird abgeschaltet.

### Figur 4M - Arbeitsphase 8

- Gebinde **9** wird von der Fördereinrichtung **6** auf drittes Transfermittel **64** geladen; und
- dritte Passage **P3** wird mittels Wegschwenken des zugehörigen Torelements **D** geöffnet, um Gebinde **9** durch dritte Passage **P3** hin zum Containment **5** auszuschieben.

### Figur 4N - Arbeitsphase 9

- Gebinde **9** ist hin zum Containment **5** ausgeschoben;
- dritte Passage **P3** als Übergang zum Containment **5** ist vom zugehörigen Torelement **D** verschlossen; und
- ein nächster Behandlungszyklus kann mit der Arbeitsphase 1 gestartet werden.

### Figur 5

Diese alternative Ausführungsform der Fördereinrichtung **6** hat einzeln schwenkbare, armförmige Stützelemente **60** zum wechselnden, temporären Ergreifen von Oberflächenarealen **942** am Gebinde **9.** Besonders vorteilhaft besitzt jedes Stützelement **60** am oberen Ende jeweils ein Bügelteil **68,** welches komplementär zum Unterfassen jeweils einer Ecke des Randes **941** des Gebindes **9** ausgebildet ist. Die unteren Enden der Stützelemente **60** stecken in einem gemeinsamen beweglichen Lager **67.**

### Figuren 6A bis 6J

Die Produktionsanlage **1** - jetzt konzipiert in *vierter Ausführungsform* - mit im Prinzip ebenfalls in der Horizontalen durchgeführtem Bewegungsverlauf der zu behandelnden Gebinde **9** und mit Verwendung der Fördereinrichtung **6** gemäss Figur 5. Insoweit gelten die sich wiederholenden Merkmale der *dritten Ausführungsform* gemäss Figuren 4A-4N, ansonsten die nachstehend beschriebenen Abwandlungen.

Die Bereitstellungsstation **2** befindet sich nun auf der Vorderseite der Prozesskammer **3,** in deren Wandung - als Bestandteil des Gehäuses **8** der Produktionsanlage **1** - die erste Passage **P1** mit zugehörigem Torelement **D** angeordnet sind. Seitlich hat die Prozesskammer **3** ihr Plenum **30** mit dem einblasenden Ventilator **301.** An der Vorderseite der Prozesskammer **3** ist die Abgangskammer **4** angebaut, welche oben das Plenum **40** mit dem einblasenden Ventilator **401** hat und durch die dritte Passage **P3** bei geöffnetem zugehörigem Torelement **D** zum Containment **5** führt. Unterhalb der Prozesskammer **3** ist die Basiskammer **35** mit ihrem seitlichen Plenum **350** und dem darinsitzenden absaugenden Ventilator **351** angeordnet. Von der Prozesskammer **3** zur Abgangskammer **4** existiert die zweite Passage **P2** mit zugehörigem Torelement **D.**

### Figur 6A - Arbeitsphase 1 (Startsituation)

- In Prozesskammer **3** und Abgangskammer **4** herrschen nach einer Initial-Dekontamination sterile Bedingungen;
- durch die Prozesskammer **3** fliesst eine gleichgerichtete Verdrängungsströmung **LF,** welche wiederum seitlich in die Prozesskammer **3** eingebracht und unten und/oder seitlich aus der Prozesskammer **3** abgeführt wird;
- erstes Transfermittel **62** in der Bereitstellungsstation **2** steht bereit zum Beladen mit einem zur Dekontaminationsbehandlung vorgesehenen Gebinde **9;**
- erste Passage **P1** und zweite Passage **P2** sind vom jeweils zugehörigen Torelement **D** verschlossen;
- dritte Passage **P2** ist durch Wegschwenken des zugehörigen Torelements **D** geöffnet;
- Fördereinrichtung **6** in der Prozesskammer **3** steht hinter erster Passage **P1** und ist unbelegt; und
- Elektronen-Strahlungsquelle **7** ist ausgeschaltet.

### Figuren 6B und 6C - Arbeitsphase X

- Fördereinrichtung **6** transportiert das zuvor durch die geöffnete erste Passage **P1** in die Prozesskammer **3** eingebrachte Gebinde **9** in einer kombinierten Seiten- und Drehbewegung durch die aus dem Austrittsfenster **70** der eingeschalteten Elektronen-Strahlungsquelle **7** emittierten Elektronenwolke; hierbei löst sich nacheinander jeweils eines der ersten Stützelemente **60** mit dem jeweiligen Bügelteil **68** vom betreffenden ergriffenen Oberflächenareal **942** am Gebinde **9,** so dass sukzessive freiliegend sämtliche Oberflächenareale **942** am Gebinde **9** von der Elektronenwolke bestrahlt werden;
- erstes Transfermittel **62** in der Bereitstellungsstation **2** steht bereit zur Aufnahme eines nächsten zur Dekontaminationsbehandlung vorgesehenem Gebindes **9;** und
- nach dem Transport des Gebindes **9** durch das Emissionsfeld der Elektronen-Strahlungsquelle **7** und bei fliessender Verdrängungsströmung **LF** ist in der Prozesskammer **3** die Sterilgrenze **5** entstanden, ab der in weiterer Verarbeitungsrichtung des Gebindes **9** Reinraumbedingungen herrschen, z.B. die Reinraumklasse A gemäss DIN EN ISO 14644-1.

### Figur 6D - Arbeitsphase X+1

- Fördereinrichtung **6** mit aufgeladenem Gebinde **9** hat Elektronenwolke durchfahren und steht in der Prozesskammer **3** vor noch vom zugehörigen Torelement **D** verschlossener zweiter Passage **P2;** und
- erstes Transfermittel **62** in der Bereitstellungsstation **2** ist mit einem nächsten zur Dekontaminationsbehandlung vorgesehenen Gebindes **9** beladen.

### Figur 6E - Arbeitsphase X+2

- Elektronen-Strahlungsquelle **7** ist ausgeschaltet; und
- zweite Passage **P2** wird durch Wegschwenken des zugehörigen Torelements **D** geöffnet.

### Figur 6F - Arbeitsphase X+3

- Die ersten Stützelemente **60** der Fördereinrichtung **6** sind vom weiterhin getragenen Gebinde **9** gelöst.

### Figur 6G - Arbeitsphase X+4

- Behandeltes Gebinde **9** ist durch zweite Passage **P2** auf drittes Transfermittel **64** in der Abgangskammer **4** geladen.

### Figur 6H - Arbeitsphase X+5

- Leere Fördereinrichtung **6** ist innerhalb der Prozesskammer **3** zurück an erste Passage **P1** gefahren;
- erste Passage **P1** wird geöffnet, zugehöriges Torelement **D** ist weggeschwenkt; und
- zweite Passage **P2** ist vom zugehörigen Torelement **D** ist geschlossen.

### Figur 6J - Arbeitsphase X+6

- Das nächste zur Dekontaminationsbehandlung vorgesehene Gebinde **9** wurde durch die offene erste Passage **P1** vom ersten Transfermittel **62** auf Fördereinrichtung **6** geladen;
- anschliessend wurde erste Passage **P1** vom zugehörigen Torelement **D** verschlossen;
- ein weiteres zur Dekontaminationsbehandlung vorgesehene Gebinde **9** ist an Bereitstellungsstation **2** angeliefert; und
- dritte Passage **P3** ist geöffnet, zugehöriges Torelement **D** ist weggeschwenkt;
- das bereits behandelte Gebinde **9** wurde vom dritten Transfermittel **64** aus der Abgangskammer **4** durch dritte Passage **P3** hin zum Containment **5** überführt; und
- die dekontaminierende Behandlung des nächsten und weiterer Gebinde **9** in der Produktionsanlage **1** kann fortgesetzt werden.

### Figuren 7A bis 7K

Die Produktionsanlage **1** in *fünfter Ausführungsform,* erweitert mit einer Eingangsschleuse **25** und mit im Prinzip ebenfalls in der Horizontalen durchgeführtem Bewegungsverlauf der zu behandelnden Gebinde **9.** Insoweit gelten die sich wiederholenden Merkmale der *vierten Ausführungsform* gemäss Figuren 6A-6J, ansonsten die nachstehend beschriebenen Abwandlungen.

Eingangsschleuse **25** und Abgangskammer **4** sind beide nebeneinander - und voneinander durch die Zwischenwand **88** abgegrenzt - der Prozesskammer **3** vorgesetzt. Von der Abgangskammer **4** geht es in das Containment **5** über und vor der Eingangsschleuse **25** ist die Bereitstellungsstation **2** angeordnet. Der Bereitstellungsstation **2** zugewandt hat die Eingangsschleuse **25** die vierte Passage **P4,** welcher ein Torelement **D** zugehörig ist und die in die Eingangsschleuse **25** führt. In der Bereitstellungsstation **2** sind das erste Transfermittel **62** und in der Eingangsschleuse **25** das fünfte Transfermittel **61** positioniert.

### Figur 7A - Arbeitsphase 1 (Startsituation)

- In Prozesskammer **3** und Abgangskammer **4** herrschen nach einer Initial-Dekontamination sterile Bedingungen;
- durch die Prozesskammer **3** fliesst eine gleichgerichtete Verdrängungsströmung **LF,** welche hier erneut von seitlich in die Prozesskammer **3** eingebracht und unten und/oder seitlich aus der Prozesskammer **3** abgeführt wird;
- erste Passage **P1**, zweite Passage **P2** und dritte Passage **P3** sind vom jeweils zugehörigen Torelement **D** verschlossen;
- vierte Passage **P4** ist durch Wegschwenken des zugehörigen Torelements **D** geöffnet;
- in der Bereitstellungsstation **2** ist erstes Transfermittel **62** mit einem zur Dekontaminationsbehandlung vorgesehenen Gebinde **9** beladen;
- Fördereinrichtung **6** in der Prozesskammer **3** steht hinter erster Passage **P1** und ist unbelegt; und
- Elektronen-Strahlungsquelle **7** ist eingeschaltet.

### Figuren 7B bis 7D - Arbeitsphase X

Zur Verkürzung von Zeichnungssatz und Beschreibung werden bis zu folgender Situation Zwischenphasen übersprungen:
- Das aus Figur 7A stammende Gebinde **9** wurde durch die vierte offene Passage **P4** in die Eingangsschleuse **25** gebracht, hier auf das fünfte Transfermittel **61** geladen und durch die erste offene Passage **P1** auf die Fördereinrichtung **6** in der Prozesskammer **3** geladen; vor dem Öffnen der ersten Passage **P1** wurde die vierte Passage **P4** mit dem zugehörigen Torelement **D** verschlossen; nach dem Einbringen Gebindes **9** in die Prozesskammer **3** wurde die erste Passage **P1** mit dem zugehörigen Torelement **D** verschlossen und darauf die vierte Passage **P4** durch Wegschwenken des zugehörigen Torelements **D** wieder geöffnet;
- anschliessend transportiert die Fördereinrichtung **6** in der Prozesskammer **3** das Gebinde **9** in einer kombinierten Seiten- und Drehbewegung durch die aus dem Austrittsfenster **70** der Elektronen-Strahlungsquelle **7** emittierten Elektronenwolke; hierbei löst sich nacheinander jeweils eines der ersten Stützelemente **60** mit dem jeweiligen Bügelteil **68** vom betreffenden ergriffenen Oberflächenareal **942** am Gebinde **9,** so dass sukzessive freiliegend sämtliche Oberflächenareale **942** am Gebinde **9** von der Elektronenwolke bestrahlt werden;
- in der Bereitstellungsstation **2** ist das erste Transfermittel **62** mit einem nächsten zur Dekontaminationsbehandlung vorgesehenen Gebinde **9** beladen; und
- nach dem Transport des Gebindes **9** durch das Emissionsfeld der Elektronen-Strahlungsquelle **7** und bei fliessender Verdrängungsströmung **LF** ist in der Prozesskammer **3** die Sterilgrenze **5** entstanden, ab der in weiterer Verarbeitungsrichtung des Gebindes **9** Reinraumbedingungen herrschen, z.B. die Reinraumklasse A gemäss DIN EN ISO 14644-1.

### Figuren 7E - Arbeitsphase X+1

- Das nächste aus den Figuren 7B-7D stammende Gebinde **9** befindet sich auf dem fünften Transfermittel **61** in der Eingangsschleuse **25;** und
- Fördereinrichtung **6** mit aufgeladenem Gebinde **9** hat Elektronenwolke durchfahren und steht in der Prozesskammer **3** vor noch vom zugehörigen Torelement **D** verschlossener zweiter Passage **P2.**

### Figuren 7F - Arbeitsphase X+2

- Zweite Passage **P2** wird durch Wegschwenken des zugehörigen Torelements **D** geöffnet; und
- vierte Passage **P4** wird vom zugehörigen Torelement **D** verschlossen.

### Figuren 7G - Arbeitsphase X+3

- Die ersten Stützelemente **60** der Fördereinrichtung **6** sind vom weiterhin getragenen Gebinde **9** gelöst.

### Figuren 7H - Arbeitsphase X+4

- Behandeltes Gebinde **9** ist durch zweite Passage **P2** auf drittes Transfermittel **64** in der Abgangskammer **4** geladen.

### Eiguren 7J - Arbeitsphase X+5

- Leere Fördereinrichtung **6** ist innerhalb der Prozesskammer **3** zurück an erste Passage **P1** gefahren;
- erste Passage **P1** und dritte Passage **P3** werden geöffnet, das jeweils zugehörige Torelement **D** ist weggeschwenkt; und
- zweite Passage **P2** ist vom zugehörigen Torelement **D** ist geschlossen.

### Figuren 7K - Arbeitsphase X+6

- Das nächste aus Figur 7J stammende Gebinde 9 wurde durch die offene erste Passage **P1** vom fünften Transfermittel **61** auf Fördereinrichtung **6** geladen;
- anschliessend wurde erste Passage **P1** vom zugehörigen Torelement **D** verschlossen;
- ein weiteres zur Dekontaminationsbehandlung vorgesehene Gebinde **9** ist an Bereitstellungsstation **2** angeliefert; und
- dritte Passage **P3** ist vom zugehörigen Torelement **D** verschlossen;
- das bereits behandelte Gebinde **9** wurde vom dritten Transfermittel **64** aus der Abgangskammer **4** durch dritte Passage **P3** hin zum Containment **5** überführt; und
- die dekontaminierende Behandlung des nächsten und weiterer Gebinde **9** in der Produktionsanlage **1** kann fortgesetzt werden.

## Patentansprüche

1. Verfahren zum seriellen Transfer von Gebinden (**9**) durch eine Prozesskammer (**3**) zur Weiterverarbeitung in einem Containment (**5**) unter aseptischen Bedingungen mittels einer in der Prozesskammer (**3**) installierten Elektronen-Strahlungsquelle (**7**), welche ein Austrittsfenster (**70**) besitzt, aus dem ein Emissionsfeld von Elektronen austritt, wobei:
a) das einzelne Gebinde (**9**) ein Behältnis (**94**) aufweist, das mit einer Abdeckung (**98**) verschlossen ist;
b) der Prozesskammer (**3**) eine Bereitstellungsstation (**2**) vorgelagert ist, die zusammen mit dem Containment (**5**) eine in einem Aufstellungsraum (**A**) errichtete Produktionsanlage (**1**) bilden;
c) aus der Bereitstellungsstation (**2**) werden die Gebinde (**9**) einzeln und nacheinander auf eine in der Prozesskammer (**3**) installierte Fördereinrichtung (**6**) geladen;
d) die Fördereinrichtung (**6**) dazu dient, das einzeln aufgenommene Gebinde (**9**) durch das wirksame Emissionsfeld der Elektronen-Strahlungsquelle (**7**) zu bewegen, **gekennzeichnet durch** die im Arbeitsmodus der Produktionsanlage (**1**) aufeinanderfolgenden Verfahrensschritte:
e) Transport des äusserlich zu dekontaminierenden Gebindes (**9**) **durch** das Emissionsfeld der Elektronen-Strahlungsquelle (**7**), während die Fördereinrichtung (**6**) das aufgenommene Gebinde (**9**) im Wechsel an verschiedenen Oberflächenarealen (**942**) stützt, so dass die gesamte Oberfläche des Gebindes (**9**) dekontaminiert ist; und
f) der Transport als kombinierte Bewegung des Gebindes (**9**) vor dem Austrittsfenster (**70**) ausgeführt werden, nämlich:
fa) als kontinuierliche oder schrittweise Vorwärtsbewegung des Gebindes (**9**) und dazu gleichzeitiger Dreh- oder Taumelbewegung um sich selbst; oder
fb) als schrittweise Vorwärtsbewegung des Gebindes (**9**) und zwischen den vorwärtsweisenden Bewegungsschritten eine Dreh- oder Taumelbewegung um sich selbst.

2. Verfahren nach Anspruch 1, **dadurch gekennzeichnet, dass** der kombinierten Bewegung des Gebindes (**9**) vor dem Austrittsfenster (**70**), bestehend aus Vorwärtsbewegung und Dreh- oder Taumelbewegung um sich selbst, eine variierende seitliche Auslenkung des Gebindes (**9**) überlagert ist, um zu erreichen, dass zumindest im Prinzip sämtliche Oberflächenareale (**942**) des Gebindes (**9**) während der wirksamen Bestrahlung durch die Elektronen-Strahlungsquelle (**7**) zum Austrittsfenster (**70**) in gleichem Abstand geführt werden.

3. Verfahren nach Anspruch 1, **dadurch gekennzeichnet, dass** die Vorwärtsbewegung geradlinig verläuft.

4. Verfahren nach zumindest einem der Ansprüche 1 bis 3, **dadurch gekennzeichnet, dass** während des Transports die Flächennormale des Austrittsfensters (**70**) und die Flächennormale der Abdeckung (**98**) zueinander einen Winkel von 90° oder einen Winkel zwischen 45° und maximal 135°, vorzugsweise zwischen 60° und maximal 120° bilden.

5. Verfahren nach zumindest einem der Ansprüche 1 bis 4, **dadurch gekennzeichnet, dass** zur Gewährleistung des Personenschutzes im Aufstellraum (**A**) gegenüber der Gefährdung, welche von der Elektronen-Strahlungsquelle (**7**) im Betriebsmodus ausgeht:
a) die Elektronen-Strahlungsquelle (**7**) in kritischen Situationen mit aus der Prozesskammer (**3**) heraus nach aussen offenem Strahlungsweg, zumindest auf eine unkritische Leistung heruntergeregelt oder völlig deaktiviert wird; oder
b) bei laufend eingeschalteter Elektronen-Strahlungsquelle (**7**) zur Abschirmung gefährlicher Strahlung der Prozesskammer (**3**) eine Eingangsschleuse (**25**) vorgesetzt ist, über welche die zu behandelnden Gebinde (**9**) von der Bereitstellungsstation (**2**) in die Prozesskammer (**3**) eingebracht werden.

6. Verfahren nach zumindest einem der Ansprüche 1 bis 5, **dadurch gekennzeichnet, dass** zur Verkettung der Prozesskammer (**3**) mit dem Containment (**5**), bei laufend eingeschalteter Elektronen-Strahlungsquelle (**7**) zur Abschirmung gefährlicher Strahlung, zwischen beiden eine Abgangskammer (**4**) angeordnet ist, über welche die in der Prozesskammer (**3**) behandelten Gebinde (**9**) zum Containment (**5**) hin ausgeschleust werden.

7. Verfahren nach zumindest einem der Ansprüche 1 bis 6, **dadurch gekennzeichnet, dass** das einzelne Gebinde (**9**) einen aseptischen Innenraum (**940**) und einen Durchlass (**95**) hat, der mit der Abdeckung (**98**) verschlossen ist, wobei im Innenraum (**940**) gelagerte Artikel (**960**) nach Öffnen der Abdeckung (**98**) im Containment (**5**) zu behandeln sind.

8. Verfahren nach zumindest einem der Ansprüche 1 bis 7, **dadurch gekennzeichnet, dass**
a) das Behältnis (**94**) z.B. ein wannenförmiges Tub ist;
b) die Abdeckung (**98**) typischerweise auf dem den Durchlass (**95**) des Behältnisses (**94**) umlaufenden Rand **(941)** versiegelt ist und so den Innenraum (**940**) des Behältnisses (**94**) in sterilem Zustand bewahrt;
c) das Behältnis (**94**) verschiedene Oberflächenareale (**942**) aufweist, die sich als Konturen zum Unterfassen oder klemmenden Ergreifen des Gebindes (**9**) eignen;
d) die Artikel (**960**) insbesondere Phiolen, Vials oder Spritzen sind; und
e) von der Abdeckung (**98**) vorzugsweise zumindest Flächenanteile aus einem semipermeablen Vliesgewebe, wie Tyvek , bestehen.

9. Verfahren nach zumindest einem der Ansprüche 1 bis 8, **dadurch gekennzeichnet, dass** die Fördereinrichtung (**6**) bewegliche Stützelemente (**60**) besitzt, welche während des Transports das Gebinde (**9**) im Wechsel an verschiedenen Oberflächenarealen (**942**) stützend, geschaltet werden, nämlich das Gebinde (**9**) unterfassen oder einklemmend greifen, z.B. am Boden des Gebindes (**9**) oder an dessen Behältnisrand **(941)** ansetzen.

10. Verfahren nach Anspruch 9, **dadurch gekennzeichnet, dass** während des Transports das jeweilige dem Austrittsfenster (**70**) zugewandte Stützelement (**60**) vom Gebinde (**9**) gelöst geschaltet wird.

11. Verfahren nach zumindest einem der Ansprüche 1 bis 10, **dadurch gekennzeichnet, dass** für die Bewegung der Fördereinrichtung (**6**) ein Transportschlitten und/oder eine Hubeinrichtung dient.

12. Verfahren nach zumindest einem der Ansprüche 1 bis 11, **dadurch gekennzeichnet, dass** die Elektronen-Strahlungsquelle (**7**) mit einer Beschleunigungsspannung zwischen 80 keV und 300 keV betrieben wird, vorzugsweise mit Beschleunigungsspannung zwischen 120 keV und 200 keV.

13. Verfahren nach zumindest einem der Ansprüche 1 bis 12, **dadurch gekennzeichnet, dass** durch die Prozesskammer (**3**) eine gleichgerichtete Verdrängungsströmung (**LF**) fliesst, welche von oben und/oder von der Seite in die Prozesskammer (**3**) eingebracht und unten und/oder seitlich aus der Prozesskammer (**3**) abgeführt wird.

14. Verfahren nach Anspruch 13, **dadurch gekennzeichnet, dass** nach dem Transport des Gebindes (**9**) durch das Emissionsfeld der Elektronen-Strahlungsquelle (**7**) und bei fliessender Verdrängungsströmung (**LF**) in der Prozesskammer (**3**) eine Sterilgrenze (**S**) erzielt wird, ab der in weiterer Prozessrichtung des Gebindes (**9**) Reinraumbedingungen herrschen, z.B. die Reinraumklasse A gemäss DIN EN ISO 14644-1.

15. Produktionsanlage (**1**) zum seriellen Transfer von Gebinden (**9**) durch eine Prozesskammer (**3**) zur Weiterverarbeitung in einem Containment (**5**) unter aseptischen Bedingungen mittels einer in der Prozesskammer (**3**) installierten Elektronen-Strahlungsquelle (**7**), welche ein Austrittsfenster (**70**) besitzt, aus dem ein Emissionsfeld von Elektronen austritt, wobei:
a) das einzelne Gebinde (**9**) ein Behältnis (**94**) aufweist, das mit einer Abdeckung (**98**) verschlossen ist;
b) der Prozesskammer (**3**) eine Bereitstellungsstation (**2**) vorgelagert ist, die zusammen mit dem Containment (**5**) die in einem Aufstellungsraum (**A**) errichtete Produktionsanlage (**1**) bilden;
c) in der Prozesskammer (**3**) sich eine Fördereinrichtung (**6**) befindet, welche dazu bestimmt ist, die Gebinde (**9**) aus der Bereitstellungsstation (**2**) einzeln und nacheinander aufzunehmen und das einzeln aufgenommene Gebinde (**9**) durch das wirksame Emissionsfeld der Elektronen-Strahlungsquelle (**7**) zu bewegen, **dadurch gekennzeichnet, dass**
d) die Fördereinrichtung (**6**) zum Transport des äusserlich zu dekontaminierenden Gebindes (**9**) durch das Emissionsfeld der Elektronen-Strahlungsquelle (**7**) dient und während des Transports die Fördereinrichtung (**6**) das aufgenommene Gebinde (**9**) im Wechsel an verschiedenen Oberflächenarealen (**942**) stützt, so dass die gesamte Oberfläche des Gebindes (**9**) dekontaminiert ist; und
e) der Transport eine kombinierte Bewegung des Gebindes (**9**) vor dem Austrittsfenster (**70**) ist, nämlich:
ea) eine kontinuierliche oder schrittweise Vorwärtsbewegung des Gebindes (**9**) und dazu gleichzeitige Dreh- oder Taumelbewegung um sich selbst; oder
eb) eine schrittweise Vorwärtsbewegung des Gebindes (**9**) und zwischen den vorwärtsweisenden Bewegungsschritten eine Dreh- oder Taumelbewegung um sich selbst.

16. Produktionsanlage (**1**) nach Anspruch 15, **dadurch gekennzeichnet, dass** der kombinierten Bewegung des Gebindes (**9**) vor dem Austrittsfenster (**70**), bestehend aus Vorwärtsbewegung und Dreh- oder Taumelbewegung um sich selbst, eine variierende seitliche Auslenkung des Gebindes (**9**) überlagert ist, um zu erreichen, dass zumindest im Prinzip sämtliche Oberflächenareale (**942**) des Gebindes (**9**) während der wirksamen Bestrahlung durch die Elektronen-Strahlungsquelle (**7**) zum Austrittsfenster (**70**) in gleichem Abstand geführt werden.

17. Produktionsanlage (**1**) nach Anspruch 15, **dadurch gekennzeichnet, dass** die Vorwärtsbewegung geradlinig ist.

18. Produktionsanlage (**1**) nach zumindest einem der Ansprüche 15 bis 17, **dadurch gekennzeichnet, dass** während des Transports die Flächennormale des Austrittsfensters (**70**) und die Flächennormale der Abdeckung (**98**) zueinander einen Winkel von 90° oder einen Winkel zwischen 45° und maximal 135°, vorzugsweise zwischen 60° und maximal 120° bilden.

19. Produktionsanlage (**1**) nach zumindest einem der Ansprüche 15 bis 18, **dadurch gekennzeichnet, dass** zur Gewährleistung des Personenschutzes im Aufstellraum (**A**) gegenüber der Gefährdung, welche von der Elektronen-Strahlungsquelle (**7**) im Betriebsmodus ausgeht:
a) die Elektronen-Strahlungsquelle (**7**) in kritischen Situationen mit aus der Prozesskammer (**3**) heraus nach aussen offenem Strahlungsweg, zumindest auf eine unkritische Leistung heruntergeregelt oder völlig deaktiviert wird; oder
b) bei laufend eingeschalteter Elektronen-Strahlungsquelle (**7**) zur Abschirmung gefährlicher Strahlung der Prozesskammer (**3**) eine Eingangsschleuse (**25**) vorgesetzt ist, über welche die zu behandelnden Gebinde (**9**) von der Bereitstellungsstation (**2**) in die Prozesskammer (**3**) eingebracht werden.

20. Produktionsanlage (**1**) nach zumindest einem der Ansprüche 15 bis 19, **dadurch gekennzeichnet, dass** zur Verkettung der Prozesskammer (**3**) mit dem Containment (**5**), bei laufend eingeschalteter Elektronen-Strahlungsquelle (**7**) zur Abschirmung gefährlicher Strahlung, zwischen beiden eine Abgangskammer (**4**) angeordnet ist, über welche die in der Prozesskammer (**3**) behandelten Gebinde (**9**) zum Containment (**5**) hin ausgeschleust werden.

21. Produktionsanlage (**1**) nach zumindest einem der Ansprüche 15 bis 20, **dadurch gekennzeichnet, dass** das einzelne Gebinde (**9**) einen aseptischen Innenraum (**940**) und einen Durchlass (**95**) hat, der mit der Abdeckung (**98**) verschlossen ist, wobei im Innenraum (**940**) gelagerte Artikel (**960**) nach Öffnen der Abdeckung (**98**) zur Behandlung im Containment (**5**) bestimmt sind.

22. Produktionsanlage (**1**) nach zumindest einem der Ansprüche 15 bis 21, **dadurch gekennzeichnet, dass**
a) das Behältnis (**94**) z.B. ein wannenförmiges Tub ist;
b) die Abdeckung (**98**) typischerweise auf dem den Durchlass (**95**) des Behältnisses (**94**) umlaufenden Rand (**941**) versiegelt ist und so den Innenraum (**940**) des Behältnisses (**94**) in sterilem Zustand bewahrt;
c) das Behältnis (**94**) verschiedene Oberflächenareale (**942**) aufweist, die sich als Konturen zum Unterfassen oder klemmenden Ergreifen des Gebindes (**9**) eignen;
d) die Artikel (**960**) insbesondere Phiolen, Vials oder Spritzen sind; und
e) von der Abdeckung (**98**) vorzugsweise zumindest Flächenanteile aus einem semipermeablen Vliesgewebe, wie Tyvek^{®}, bestehen.

23. Produktionsanlage (**1**) nach zumindest einem der Ansprüche 15 bis 22, **dadurch gekennzeichnet, dass** die Fördereinrichtung (**6**) bewegliche Stützelemente (**60**) besitzt, welche dazu bestimmt sind, sich während des Transports schalten zu lassen, um das das Gebinde (**9**) im Wechsel an verschiedenen Oberflächenarealen (**942**) zu stützen, nämlich das Gebinde (**9**) unterfassen oder einklemmend greifen, z.B. am Boden des Gebindes (**9**) oder an dessen Behältnisrand (**941**) ansetzen.

24. Produktionsanlage (**1**) nach Anspruch 23, **dadurch gekennzeichnet, dass** während des Transports das jeweilige dem Austrittsfenster (**70**) zugewandte Stützelement (**60**) vom Gebinde (**9**) gelöst schaltbar ist.

25. Produktionsanlage (**1**) nach zumindest einem der Ansprüche 15 bis 24, **dadurch gekennzeichnet, dass** für die Bewegung der Fördereinrichtung (**6**) ein Transportschlitten und/oder eine Hubeinrichtung dient.

26. Produktionsanlage (**1**) nach zumindest einem der Ansprüche 15 bis 25, **dadurch gekennzeichnet, dass** die Elektronen-Strahlungsquelle (**7**) mit einer Beschleunigungsspannung zwischen 80 keV und 300 keV betrieben wird, vorzugsweise mit Beschleunigungsspannung zwischen 120 keV und 200 keV.

27. Produktionsanlage (**1**) nach zumindest einem der Ansprüche 15 bis 26, **dadurch gekennzeichnet, dass** durch die Prozesskammer (**3**) eine gleichgerichtete Verdrängungsströmung (**LF**) fliesst, welche von oben und/oder von der Seite in die Prozesskammer (**3**) eingebracht und unten und/oder seitlich aus der Prozesskammer (**3**) abgeführt wird.

28. Produktionsanlage (**1**) nach Anspruch 27, **dadurch gekennzeichnet, dass** nach dem Transport des Gebindes (**9**) durch das Emissionsfeld der Elektronen-Strahlungsquelle (**7**) und bei fliessender Verdrängungsströmung (**LF**) in der Prozesskammer (**3**) eine Sterilgrenze (**S**) entstanden ist, ab der in weiterer Verarbeitungsrichtung des Gebindes (**9**) Reinraumbedingungen herrschen, z.B. die Reinraumklasse A gemäss DIN EN ISO 14644-1.
